# NEUE EUROPÄISCHE PATENTSCHRIFT

(11) **EP 2 705 020 B2**
(45) Veröffentlichungstag und Bekanntmachung des Hinweises auf die Entscheidung über den Einspruch: **05.11.2025**
(45) Hinweis auf die Patenterteilung: 08.04.2015
(21) Anmeldenummer: 12723831.9
(22) Anmeldetag: 18.05.2012
(51) Int. Cl.: C07C 201/16, C07C 205/06, B01J 14/00

(54) **VERFAHREN UND VORRICHTUNG ZUR AUFREINIGUNG VON NITRIERPRODUKTEN**
METHOD AND APPARATUS FOR PURIFYING NITRATION PRODUCTS
PROCÉDÉ ET DISPOSITIF DE PURIFICATION DE PRODUITS DE NITRATION

(30) Priorität: 19.05.2011 DE 102011102059
(43) Veröffentlichungstag der Anmeldung: 12.03.2014
(60) Teilanmeldung: 13005208.7
(73) Patentinhaber: Josef Meissner GmbH & Co. KG, 50968 Köln (DE)
(72) Erfinder: PÖHLMANN, Jürgen, 50969 Köln (DE); HERMANN, Heinrich, 50858 Köln (DE); HÄNDEL, Mirko, 53819 Neunkirchen-Seelscheid (DE); GEBAUER, Jürgen, 53840 Troisdorf (DE)
(74) Vertreter: Von Rohr Patentanwälte Partnerschaft mbB
(86) Internationale Anmeldenummer: PCT/EP2012/002139
(87) Internationale Veröffentlichungsnummer: WO 2012/156095

(56) Entgegenhaltungen:
- EP-A2- 0 279 312
- CN-A- 1 597 557
- CN-Y- 2 729 072
- DE-A1- 2 151 206
- DE-B- 1 222 904
- US-A- 3 221 064
- US-A- 4 482 769
- US-A- 4 597 875

## Beschreibung

Die vorliegende Erfindung betrifft das technische Gebiet der Nitrierung, insbesondere der Herstellung nitrierter aromatischer organischer Verbindungen (synonym nachfolgend auch als "Nitroaromaten", "Nitrierprodukte" oder dergleichen bezeichnet) und deren Aufreinigung nach ihrer Herstellung.

Insbesondere betrifft die vorliegende Erfindung ein Verfahren zur Entfernung von Verunreinigungen (wie z. B. insbesondere nichtumgesetzten Edukten, Reaktionsnebenprodukten, Nitriersäure und deren Umsetzungsprodukten, wie Stickoxiden oder Salpetriger Säure etc.) aus bei der Nitrierung von nitrierbaren aromatischen Verbindungen nach Abtrennung der Nitrierendsäure anfallenden nitrierten Rohprodukten durch Behandlung mit einem Waschmedium. Mit anderen Worten betrifft die vorliegende Erfindung somit ein Verfahren zur Aufreinigung von bei der Nitrierung von nitrierbaren aromatischen Verbindungen nach Abtrennung der Nitrierendsäure anfallenden nitrierten Rohprodukten.

Schließlich betrifft die vorliegende Erfindung eine Produktionsanlage zur Nitrierung nitrierbarer aromatischer Verbindungen mit nachfolgender Aufreinigung der nitrierten Produkte.

Aromatische Nitroverbindungen, wie z. B. Nitrobenzol (MNB), Mononitrotoluol (MNT), Dinitrotoluol (DNT), Trinitrotoluol (TNT), Nitrochlorbenzol (MNCB) etc., welche durch Umsetzung eines entsprechenden Aromaten, wie z. B. Benzol, Toluol, Xylol, Chlorbenzol, Dichlorbenzolen etc., mit Salpetersäure - direkt oder in Gegenwart von Schwefelsäure als Katalysator und wasserbindendes Mittel - hergestellt werden, müssen vor ihrer Weiterverarbeitung einer mehrstufigen Wäsche und zusätzlichen Reinigungsstufen unterworfen werden, um die in den rohen Nitroaromaten noch gelösten oder suspendierten Verunreinigungen, wie z. B. Schwefelsäure, Salpetersäure, Nitrose, Nitrophenole, Nitrokresolen etc., die beispielsweise als Mono-, Dinitro- und Trinitroverbindungen vorliegen können, und sonstige Oxidationsprodukte, wie z. B. Nitrobenzoesäuren und Abbauprodukte aus der Zersetzung der Nitrophenole, bzw. die nichtumgesetzten Aromaten oder nicht gewünschte Isomere, wie z. B. bei der TNT-Herstellung, im rohen Gemisch der Nitroaromaten zu entfernen.

Üblicherweise besteht die Wäsche der rohen Nitroaromaten zur Entfernung der darin gelösten und suspendierten Säuren des Nitriergermisches, der Nitrophenole und anderer saurer und sonst noch mit dem Waschmittel extrahierbaren Verunreinigungen aus drei Schritten (siehe z. B. F. Meissner et al., Industrial and Engineering Chemistry, Vol. 46, Seiten 718 bis 724 (1954); Ullmanns Enzyklopädie der Technischen Chemie, 4. Auflage, Bd. 17, Seiten 384 bis 386; H. Hermann et al., "Industrial Nitration of Toluene to Dinitrotoluene", ACS Symposium Series 623 (1996), Seiten 234 bis 249, Editors: L. F. Albright, R. V. C. Carr, R. J. Schmitt; US 6 288 289 B1; EP 1 816 117 B1). Als Waschmedium wird dafür üblicherweise Wasser eingesetzt, wobei die Wäsche üblicherweise als flüssig/flüssig-Wäsche (d. h. bei Temperaturen, bei denen der zu waschende Nitroaromat als Flüssigkeit vorliegt) durchgeführt wird.

Diese dreistufige Wäsche umfasst üblicherweise die folgenden Schritte:
1. Eine saure Wäsche mit Wasser zur Entfernung der gelösten und suspendierten Mineralsäuren, wie z. B. Schwefelsäure, Salpetersäure und Nitrose ("saure Wäsche").
2. Eine basische bzw. alkalische Wäsche in Gegenwart einer Base ("Alkaliwäsche"), wie z. B. Natriumcarbonat (Soda), Natriumbicarbonat, Natriumsulfit, Natriumhydrogensulfit, Ammoniak, Natronlauge, Kalilauge etc. (siehe z. B. US 4 482 769 A, US 4 597 875 A oder US 6 288 289 B1), zur Entfernung der im rohen Nitroaromaten gelösten schwach aciden Verunreinigungen, wie der Nitrophenole, Nitrokresole, Nitrobenzoesäuren, Abbauprodukte aus der oxidativen Zersetzung der Phenole oder von aliphatischen oder cyclischen Kohlenwasserstoffen etc., wie z. B. Oxalsäure etc., oder den asymmetrischen Isomeren beim TNT ("basische Wäsche").
3. Eine Neutralwäsche zur Entfernung der Restspuren von Alkali und der weiteren Reduzierung der noch in Spuren im Produkt verbliebenen Verunreinigungen ("neutrale Wäsche").

Ziel dieser Waschschritte ist es, neben einem reinen Produkt möglichst wenig Abwasser pro Tonne Produkt zu erhalten, in welchem die ausgewaschenen Verunreinigungen so vorliegen, dass ihre Entsorgung kostengünstig durchgeführt werden kann.

Zur Minimierung der für diese Wäsche benötigten Wassermengen kann die Wäsche beispielsweise im Gegenstrom derart erfolgen, dass das zur Neutralwäsche benutzte Wasser nach Zusatz von Basen in der Alkaliwäsche eingesetzt wird (vgl. z. B. A. B. Quakenbush et. al., The Olin Dinitrotoluene (DNT) Process, Polyurethanes World Congress 1993, Publish.: Technomic Lancaster, Seiten 484 bis 488) oder aber dass bei der sauren Wäsche mit einer minimalen Menge an Wasser derart gewaschen wird, dass eine konzentrierte Säure erhalten wird, welche direkt oder nach weiterer Aufkonzentrierung in die Nitrierung zurückgeführt werden kann.

So werden in der EP 0 279 312 B1, der EP 0 736 514 B1 und der EP 1 780 195 B1 Verfahren beschrieben, mit denen die in den Nitroaromaten nach der Nitrierung noch suspendierten und gelösten Mineralsäuren, wie Schwefelsäure, Salpetersäure und Nitrose, mehrstufig und selektiv ausgewaschen und in die Nitrierung zurückgeführt werden, so dass kein Abwasser mehr aus der sauren Wäsche anfällt und entsorgt werden muss.

Es sind aber auch Verfahren bekannt geworden, bei denen - um die zu behandelnde Abwassermenge zu minimieren - keine saure Wäsche durchgeführt wird, sondern nur eine alkalische und Neutralwäsche, wie beispielsweise in Kirk-Othmer, Encyclopedia of Chemical Technology, 4. Ed., Vol. 17, Seiten 136 bis 138, oder in der US 4 091 042 A beschrieben.

Neben der Minimierung der Abfallströme ist es weiterhin das Ziel, den für die Wäsche benötigten technischen Aufwand zu minimieren (z. B. dadurch, dass die für die Wäschen eingesetzte Technologie nicht nur an die Waschstufe, sondern auch an das zu waschende Produkt spezifisch angepasst wird).

Als Waschapparate werden für die Wäsche der zu reinigenden Nitroaromaten auf den einzelnen Waschstufen üblicherweise so genannte Mixer-Settler (Mischer-Scheider) (vgl. z. B. EP 1 593 654 A1) eingesetzt, bei denen der Mischteil üblicherweise ein Rührkessel ist (vgl. z. B. Ullmann's Encyclopedia of Industrial Chemistry, 5. Ed., Vol. B 3, Seiten 6.19 bis 6.21; M. Baerns et al., Technische Chemie, Verlag Wiley-VCH 2006, Seiten 352/352). So wird bereits in der deutschen Patentschrift DE 1 135 425 eine Anordnung von Mischer und Scheider beschrieben, die es erlaubt, auch bei Raumtemperatur kristalline Nitroaromaten, wie z. B. DNT, TNT oder NCB, bei erhöhten Temperaturen unter Minimierung des Beheizungsaufwandes flüssig zu waschen. Aber auch Kreiselpumpen und statische Mischer sind als Mischer eingesetzt worden (vgl. z. B. die Druckschriften US 3 221 064 A oder EP 1 816 117 B1)

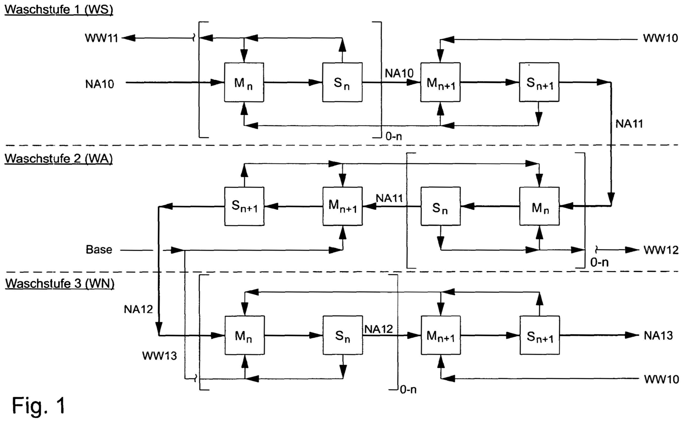

Der Einsatz der Mixer/Settler-Technologie (Mischer/Scheider-Technologie) (vgl. z. B. Fig. 1) ist aber aufwendig und teuer. Durch den nicht vermeidbaren Schlupf bei kontinuierlich betriebenen Rührkesseln als Mixer bzw. Mischer ist es vor allem bei der Entfernung der Nitrophenole oder Nitrokresole, wenn diese in hohen Konzentrationen im rohen Nitroaromaten vorliegen, notwendig, mehrstufig und möglichst im Gegenstrom zu arbeiten, um den für die Weiterverarbeitung des Nitroaromaten gewünschten niedrigen Gehalt an Verunreinigungen zu erhalten (z. B. einen Gehalt an Nitrophenolen von weniger als 10 ppm, bevorzugt 2 bis 3 ppm). Auch eine Wäsche in mehrstufigen Extraktionskolonnen ist technisch aufwendig und teuer und nicht sehr effektiv. Außerdem ist die Erzeugung großer Austauschflächen für ein zweiphasiges Gemisch in kurzer Zeit für einen effektiven Massentransfer ("*mass transfer*"), gefolgt von einer schnellen chemischen Umsetzung, weder in einem Rührkessel noch in Extraktionskolonnen realisierbar.

In J. M. Coulson, F. E. Warner, "A Problem in Chemical Engineering Design: The Manufacture of Mononitrotoluene", einer Veröffentlichung von "The Institution of Chemical Engineers", 56, Victoria Street, London, S.W. I, 1949, Seiten 25/26, wird eine dreifache Wäsche des MNT mit einem Wäscher von Typ Holley-Mott (Mixer/Settler) beschrieben, wobei die saure und die alkalische Wäsche in jeweils mindestens zwei Stufen im Gegenstrom durchgeführt wird, um eine ausreichende Entfernung der im MNT gelösten bzw. suspendierten Säuren und Nitrokresole zu erreichen.

In der kanadischen Patentschrift CA 1 034 603 wird eine vierstufige saure Wäsche im Gegenstrom vorgeschlagen, um die im rohen DNT gelöste und suspendierte Salpeter- und Schwefelsäure auszuwaschen.

In der US 4 091 042 A wird zur Entfernung aller saurer Komponenten aus rohem Nitrobenzol, wie mitgerissener Schwefelsäure und der im Nitroaromaten gelösten Dinitrophenole und Pikrinsäure von bis zu 2.000 ppm, eine vierstufige Wäsche mit Soda im Gegenstrom beschrieben, um die gewünschte Reinheit zu erhalten.

In der EP 1 816 117 A1 wird eine vierstufige Neutralwäsche im Gegenstrom unter Einsatz von vier Rührwerksbehältern und den zugehörigen Trennapparaten beschrieben (sogenannte "Mixer/Settler-Technologie"), um den noch zu hohen Gehalt an Nitrophenolen nach der alkalischen Wäsche von ca. 50 ppm auf einen Restgehalt von ca. 2 ppm zu reduzieren. Aber auch bei Ersatz der Rührwerksbehälter durch Kreiselpumpen als Mischorgan werden immer noch drei Stufen benötigt, um einen Restgehalt an Nitrophenolen im resultierenden Nitrobenzol von 3 ppm zu erhalten.

Die US 4 994 242 A offenbart, dass statische Mischer im technischen Maßstab allein als Mischorgan in zweiphasigen Systemen nicht geeignet sind, um eine optimale Dispergierung der zwei nicht miteinander mischbaren Phasen ineinander zu erzeugen. So wird in der EP 1 816 117 B1 der Einsatz eines statischen Mischers für die alkalische Wäsche beschrieben; das damit behandelte Nitrobenzol enthält noch mehr als 50 ppm an Nitrophenolen, die durch eine aufwendige mehrstufige Neutralwäsche auf ca. 2 ppm reduziert werden müssen.

Wie bereits in der EP 1 780 195 B1 für eine saure Wäsche erläutert, ist die Wäsche von Nitroaromaten ein komplexer Vorgang. Neben der Erzeugung einer genügend großen Austauschfläche zwischen Organphase und Waschphase (üblicherweise Wasser), um einen optimalen Übergang des aus der Organphase zu entfernenden Verunreinigung zu erreichen, hängt die Effektivität einer Waschstufe von den Verteilungsgleichgewichten der Verunreinigung zwischen Organphase und Waschmedium und auch davon ab, ob die aus der Organphase extrahierte Verunreinigung als solche in dem Waschmedium stabil ist oder durch eine nachfolgende Umsetzung dem Verteilungsgleichgewicht entzogen wird.

So reagiert Nitrose nach dem Übergang aus der Organphase in die wässrige Phase mit Wasser unter Disproportionierung zu Salpetersäure und NO nach Gleichung (1):

(1) 3 NO₂ (= 3/2 N₂O₄) + H₂O → 2 HNO₃ + NO

Sowohl der Übergang der Nitrose aus der Organphase, wahrscheinlich als Dimeres, als auch die Umsetzung der Nitrose (als N₂O₄) mit dem Wasser sind vergleichsweise langsam ablaufende Reaktionen im Vergleich zu einer Neutralisation, so dass für die Entfernung der Nitrose aus der Organphase durch eine Wäsche mit nachfolgender chemischer Umsetzung Zeit benötigt wird.

Bei Säuren hingegen, wie Schwefelsäure, Salpetersäure oder den schwach sauren Nitrophenolen, ist die im Waschwasser eintretende Dissoziation der Säuren in Hydroniumionen und die zugehörigen Anionen (Gleichung 2) oder die in Gegenwart von Alkali erfolgende Neutralisation (Gleichung 3) ein sehr schneller Vorgang, durch welchen die ausgewaschenen Verunreinigungen dem Verteilungsgleichgewicht zwischen Nitroaromaten und Waschwasser entzogen werden und sich in anionischer Form nur noch im Waschwasser befinden.

(2) H₂SO₄ + H₂O → H₃O⁺ + HSO₄⁻

(3) NO₂Ar-OH + NaOH → NO₂Ar-O⁻ Na⁺+ H₂O

Durch diese schnelle Neutralisation von Anionen bildenden Stoffen im alkalischen Waschmedium ist zu erwarten, dass die Extraktion dieser Stoffe aus der Organphase im Wesentlichen massentransferkontrolliert verläuft und die Wäsche im Wesentlichen den gleichen kinetischen Gesetzmäßigkeiten folgt wie eine Mononitrierung, z. B. wie die Nitrierung von Benzol zu Nitrobenzol.

Das Dokument DE 1 222 904 A betrifft ein Verfahren zur Reinigung von nitrophenolhaltigen aromatischen Nitroverbindungen, wobei die zu reinigenden aromatischen Nitroverbindungen einer Wäsche mit Wasser unterzogen und nachfolgend entweder unmittelbar oder nach Vermischung mit dem gleichen Volumen einer 0,1- bis 2gewichtsprozentige wässrigen Natronlauge versetzt und anschließend über ein alkalisch gestelltes Anionenaustauscherharz geleitet werden.

Weiterhin betrifft Dokument US 3 221 064 A ein Verfahren zur Reinigung von Dinitrotoluolen, wobei die Dinitrotoluole in einer Kreiselpumpe mit Wasser sowie wässriger Natriumhydroxidlösung emulgiert werden und nach jedem Waschschritt die Abtrennung des entsprechenden Waschmediums erfolgt.

Zudem betrifft das Dokument EP 0 279 312 A2 ein Verfahren zur Abtrennung von Schwefelsäure und Salpetersäure aus bei der Nitrierung von Toluol erhaltenen Dinitrotoluolgemischen, wobei die Dinitrotoluole mit Wasser vermischt und die sich danach abscheidende Schwefel- und Salpetersäure enthaltende wässrige Phase abgetrennt wird.

Darüber hinaus betrifft das Dokument US 4 597 875 A ein Verfahren zur Entfernung von aus bei der Herstellung von Nitroaromaten anfallenden Nitrokresol- und Pikrinsäureverunreinigungen aus Abwässern, wobei die Verunreinigungen über mehrere Waschschritte kumuliert, mit Säure behandelt und anschließend die ausgefällten organischen Rückstände verbrannt werden.

Weiterhin betrifft das Dokument US 4 482 769 A ein Verfahren zur Reinigung von Dinitrotoluol, wobei das Dinitrotoluol mit einer wässrigen basischen Phase gewaschen wird, um die Trinitroorthokresol-Verbindungen zu entfernen, das Dinitroorthokresol jedoch in der organischen Phase zu belassen.

Schließlich betrifft Dokument DE 2 151 206 A ein Verfahren und eine Vorrichtung zum Vermischen von Flüssigkeiten, wobei die Vermischung von Flüssigkeiten unter Verwendung von Strahldüsen durchgeführt wird, welche in einen Mischreaktor hineinragen.

Die aus dem Stand der Technik bekannten Verfahren und Anlagen zur Aufreinigung nitrierter Rohprodukte arbeiten oftmals nicht mit hoher Effizienz oder aber nicht in zufriedenstellender Weise. Bisweilen sind hiermit auch übermäßig komplexe Verfahrensabläufe oder Arbeitsgänge verbunden, und es werden oftmals nicht die gewünschten Reinheiten erreicht, zumindest nicht mit vertretbarem Aufwand.

Der vorliegenden Erfindung liegt daher die Aufgabe zugrunde, ein Verfahren zur Entfernung von Verunreinigungen aus bei der Nitrierung von nitrierbaren aromatischen Verbindungen nach Abtrennung der Nitrierendsäure anfallenden nitrierten Rohprodukten bereitzustellen, wobei die zuvor geschilderten, im Zusammenhang mit dem Stand der Technik auftretenden Probleme und Nachteile zumindest weitgehend vermieden oder aber wenigstens abgeschwächt werden sollen.

Insbesondere ist eine Aufgabe der vorliegenden Erfindung darin zu sehen, ein Verfahren bereitzustellen, mit dem eine effiziente Aufreinigung der nitrierten Rohprodukte, wie sie aus der Nitrierung von nitrierbaren aromatischen Verbindungen nach Abtrennung der so genannten Nitrierendsäuren hervorgehen, ermöglicht werden sollen.

Weiterhin besteht eine Aufgabe der vorliegenden Erfindung darin, die Wäsche der nach Abtrennung der Nitrierendsäure resultierenden rohen Nitroaromaten, in denen signifikante Mengen an Verunreinigungen, wie beispielsweise noch mitgerissene Nitriersäure, gelöste Schwefelsäure, Salpetersäure, Nitrose, Nitrophenole, Nitrobenzoesäuren, Abbauprodukte aus dem oxidativen Abbau von Nitrophenolen etc., vorliegen können, quasi einstufig in jedem Waschschritt derart durchzuführen, dass im gewaschenen Nitroaromaten der Nitrophenolgehalt möglichst gering ist (z. B. bei Nitrobenzol aus einer adiabatischen Nitrierung mit ursprünglich ca. 2.000 ppm Di- und Trinitrophenolen der Gehalt an Nitrophenolen nach der alkalischen Wäsche unter 50 ppm, bevorzugt unter 10 ppm, und nach der Neutralwäsche unter 2 ppm liegt) und dass der Aufwand und die Kosten dafür deutlich geringer sind als bei den bisher genutzten Verfahren des Standes der Technik.

Die zuvor geschilderte Aufgabenstellung wird erfindungsgemäß durch ein Verfahren nach Anspruch 1 gelöst; weitere, vorteilhafte Weiterbildungen und Ausgestaltungen des erfindungsgemäßen Verfahrens sind Gegenstand der diesbezüglichen Unteransprüche.

Darüber hinaus ist Gegenstand der vorliegenden Erfindung eine Produktionsanlage gemäß Anspruch 11; weitere, vorteilhafte Ausgestaltungen und Weiterbildungen dieses Aspekts sind Gegenstand der diesbezüglichen Unteransprüche.

Es versteht sich von selbst, dass Ausgestaltungen, Ausführungsformen, Vorteile oder dergleichen, welche nachfolgend - zu Zwecken der Vermeidung von unnötigen Wiederholungen - nur zu einem Erfindungsaspekt ausgeführt werden, selbstverständlich auch in Bezug auf die übrigen Erfindungsaspekte entsprechend gelten.

Zudem gilt, dass alle im Folgenden genannten Werte- bzw. Parameterangaben oder dergleichen grundsätzlich mit genormten bzw. standardisierten oder explizit angegebenen Bestimmungsverfahren oder aber mit dem Fachmann auf diesem Gebiet an sich geläufigen Bestimmungsmethoden ermittelt bzw. bestimmt werden können.

Dies vorausgeschickt, wird im Folgenden die vorliegende Erfindung näher beschrieben.

Gegenstand der vorliegenden Erfindung - gemäß einem **ersten** Aspekt der vorliegenden Erfindung - ist somit ein Verfahren zur Entfernung von Verunreinigungen aus bei der Nitrierung von nitrierbaren aromatischen Verbindungen nach Abtrennung der Nitrierendsäure anfallenden nitrierten Rohprodukten durch Behandlung mit einem Waschmedium,
**dadurch gekennzeichnet,**
dass (a) zunächst die nitrierten Rohprodukte mit einem Waschmedium in Kontakt gebracht werden und die nitrierten Rohprodukte und das Waschmedium ineinander verteilt werden derart, dass eine Emulsion resultiert, wobei die Herstellung der Emulsion in Schritt (a) mittels einer Dispergiereinrichtung erfolgt, und
dass (b) nachfolgend die resultierende Emulsion in einen Rohrreaktor einspeist wird, so dass während des Durchtritts der Emulsion durch den Rohrreaktor die in den nitrierten Rohprodukten anfänglich vorhandenen Verunreinigungen entfernt werden und/oder so dass während des Durchtritts der Emulsion durch den Rohrreaktor die in den nitrierten Rohprodukten anfänglich vorhandenen Verunreinigungen in das Waschmedium überführt und/oder hierdurch neutralisiert werden, wobei der Rohrreaktor mit Mischelementen zum Eintrag von zusätzlicher Mischenergie ausgestattet ist, wobei die Mischelemente als Bleche, als Blenden, als statische Mischer oder als Stromteiler ausgebildet sind, wobei der Druckabfall pro Mischelement 0,1 bar bis 3,0 bar beträgt.

Das erfindungsgemäße Verfahren eignet sich somit in hervorragender Weise zur Aufreinigung von bei der Nitrierung von nitrierbaren aromatischen Verbindungen nach Abtrennung der Nitrierendsäure anfallenden nitrierten Rohprodukten.

Das Prinzip des erfindungsgemäßen Verfahrens besteht also darin, die aus der Nitrierung stammenden, noch signifikante Mengen an Verunreinigungen enthaltenden rohen Nitroaromaten - nach Abtrennung der Nitrierendsäure (z. B. in einem Scheider) - zunächst mit einem Waschmedium in Kontakt zu bringen und die Mischung aus aufzureinigenden Nitroaromaten und Waschmedium in eine Emulsion bzw. Dispersion zu überführen und nachfolgend die resultierende Emulsion bzw. Dispersion in einen Rohrreaktor einzuspeisen, damit die in den aufzureinigenden Nitroaromaten anfänglich vorhandenen Verunreinigungen in das Waschmedium überführt bzw. hierdurch neutralisiert werden und auf diese Weise ein aufgereinigter Nitroaromat entsteht.

Denn, wie die Anmelderin in vollkommen überraschender Weise herausgefunden hat, führt die Verwendung eines Rohrreaktors - in Kombination mit einer vorgeschalteten Dispergier- bzw. Emulgiereinrichtung - dazu, dass eine besonders gute Durchmischung und besonders innige und feine Verteilung von Waschmedium einerseits und aufzureinigenden Nitroaromaten andererseits erreicht werden kann, so dass auf diese Weise die Verunreinigungen sozusagen in einem einzigen Verfahrensschritt (nämlich im Rahmen der Rohrreaktorbehandlung) vollständig bzw. zumindest im Wesentlichen vollständig entfernt werden können.

Im Unterschied zum Stand der Technik werden also weitergehende, komplexe Verfahrensschritte zur Aufreinigung des rohen Nitroaromaten in effizienter Weise vermieden, ohne dass Qualitätseinbußen bei der Aufreinigung des rohen Nitroaromaten hinzunehmen wären.

Überraschenderweise gewährleistet der erfindungsgemäß für die Behandlung des rohen Nitroaromaten mit dem Waschmedium eingesetzte Rohrreaktor eine derart innige und feine Verteilung von rohem Nitroaromaten einerseits und Waschmedium andererseits, dass im Rahmen der Rohrreaktorbehandlung gemäß Verfahrensschritt (b) sämtliche bzw. zumindest im Wesentlichen sämtliche Verunreinigungen in das Waschmedium überführt bzw. hierdurch neutralisiert werden, so dass sie anschließend (d. h. nach Abschluss des Verfahrensschritts (b)) zusammen mit dem Waschmedium von dem dann aufgereinigtem Nitroaromaten abgetrennt werden können.

Es hat sich nämlich in überraschender Weise gezeigt, dass es im Rahmen der vorliegenden Erfindung möglich ist, eine Wäsche von Nitroaromaten erfolgreich quasi einstufig - auch bei hoher Belastung mit Verunreinigungen, wie Nitriersäure, Nitrophenolen und Nitrokresolen - durchzuführen, und zwar durch eine einfache und kostengünstige Kombination von Strahlmischern, aber auch anderen Dispergierorganen, wie z. B. Kreiselpumpen, mit zusätzlichen Einrichtungen, wie statischen Mischern, Blenden etc. in Rohrreaktoren allein oder noch in Verbindung mit Rührkesseln, die es gestatten, eine genau definierte Mischenergie in das Gemisch der nicht miteinander mischbaren Phasen einzutragen. Die dadurch herstellbaren Emulsionen aus der zu reinigenden Organphase im Waschmedium (O/W-Typ) oder des Waschmediums in der Organphase (W/O-Typ) liefern die für eine effektiven und optimalen Massentransfer benötigten Grenzfläche zwischen zu waschendem Nitroaromat und Waschmedium.

Was die Herstellung der Emulsion bzw. Dispersion in Verfahrensschritt (a) anbelangt, so erfolgt diese erfindungsgemäß mittels einer geeigneten Dispergier- bzw. Emulgiereinrichtung, insbesondere mittels eines geeigneten Mischorgans.

Im Rahmen der vorliegenden Erfindung kann als Dispergier- bzw. Emulgiereinrichtung (d. h. insbesondere als vorzugsweise erste Dispergier- bzw. Emulgiereinrichtung), insbesondere als Mischorgan, beispielsweise ein Rührkessel, ein Strahlmischer (Jet-Mixer bzw. *Jet-Mixing-Device)* oder eine Pumpe, insbesondere eine Kreiselpumpe, eingesetzt werden.

Gemäß einer erfindungsgemäßen Ausführungsform wird als Dispergier- bzw. Emulgiereinrichtung, insbesondere als Mischorgan, eine Pumpe, insbesondere eine Kreiselpumpe, im Rahmen von Verfahrensschritt (a) eingesetzt.

Gemäß einer alternativen, erfindungsgemäß bevorzugten Ausführungsform wird im Rahmen von Verfahrensschritt (a) als Dispergier- bzw. Emulgiereinrichtung, insbesondere als Mischorgan, ein so genannter Strahlmischer (synonym auch als "Jet-Mixer" oder als "*Jet-Mixing-Device*" bezeichnet) eingesetzt. Bei dem erfindungsgemäß eingesetzten Strahlmischer handelt es sich insbesondere um eine Einrichtung, welche einen (zentralen) Treibstahl in einem den (zentralen) Treibstrahl umgebenden Medium (z. B. Ringstrahl) erzeugt.

Als Strahlmischer können alle Arten von Strahlmischern eingesetzt werden, die es gestatten, mittels des zentralen Treibstrahls als Freistrahl, der sowohl aus dem Waschmedium wie auch dem zu waschenden Nitroaromaten bestehen kann, mit hoher Relativgeschwindigkeit den zu waschenden Nitroaromaten oder das Waschmedium derart einzudüsen, dass entweder der zu waschende Nitroaromat im Waschmedium oder das Waschmedium im zu waschenden Nitroaromaten als Emulsion mit großer Grenzfläche verteilt wird. Einrichtungen dieser Art sind beispielsweise in Ullmann's Encyclopedia of Industrial Chemistry, 2003, 5. Ed., Vol. B 4, Seiten 87/88 und 565 bis 571, oder aber in Perry's Chemical Engineers' Handbook, McGraw-Hill Book Company, 1984, 6. Auflage, Seiten 5-21 bis 5-23 oder aber in der deutschen Offenlegungsschrift DE 2 151 206 beschrieben.

Dabei kann der (zentrale) Treibstrahl im Strahlmischer das Waschmedium und das umgebende Medium der aufzureinigende nitrierte Roharomat sein; alternativ kann aber auch der (zentrale) Treibstrahl durch das aufzureinigende nitrierte Rohprodukt und das den (zentralen) Treibstrahl umgebende Medium durch das Waschmedium gebildet sein. Beide alternativen Ausführungsformen führen zu dem gewünschten Ergebnis.

Besonders gute Ergebnisse im Hinblick auf die Aufreinigung des aufzureinigenden Roharomaten werden (unabhängig davon, ob der zentrale Treibstrahl durch das Waschmedium oder aber durch das aufzureinigende nitrierte Rohprodukt gebildet wird) erhalten, wenn das Verhältnis der Geschwindigkeiten zwischen dem zentralen Treibstrahl einerseits und dem den zentralen Treibstrahl umgebendem Medium (z. B. Ringstrahl) im Strahlmischer im Bereich von 1 : 5 bis 30 : 1, bevorzugt im Bereich von 1 : 2 bis 20 : 1, besonders bevorzugt im Bereich von 1 : 1 bis 10 : 1, eingestellt wird. Auf diese Weise wird eine besonders innige und feine Verteilung von Waschmedium einerseits und Rohprodukt andererseits und folglich eine besonders effiziente Aufreinigung erzielt.

Die Fließgeschwindigkeit der Waschemulsion nach dem Strahlmischer im nachfolgenden Rohrreaktor liegt insbesondere im Bereich von 0,1 bis 15,0 m/s, bevorzugt im Bereich von 0,5 bis 10 m/s.

Gemäß einer Ausführungsform der vorliegenden Erfindung kann es vorgesehen sein, dass die im Verfahrensschritt (a) eingesetzte Dispergiereinrichtung, insbesondere das Mischorgan, dem Rohrreaktor vorgeschaltet, insbesondere unmittelbar vorgeschaltet, ist. Gemäß einer besonderen Ausgestaltung dieser Ausführungsform kann es vorgesehen sein, dass die Dispergier- bzw. Emulgiereinrichtung, insbesondere das Mischorgan, in den Rohrreaktor übergeht.

Gleichermaßen ist es jedoch auch möglich, dass die Dispergiereinrichtung, insbesondere das Mischorgan, in den Rohrreaktor integriert ist bzw. Bestandteil des Rohrreaktors ist. Zu diesem Zweck kann beispielsweise die Dispergiereinrichtung in dem oberen bzw. stromaufwärts befindlichen Teil des Rohrreaktors angeordnet sein. Eine solche Ausführungsform ist insbesondere dann möglich, wenn die Dispergiereinrichtung, insbesondere das Mischorgan, als so genannter Strahlmischer ausgebildet ist.

Gemäß der Erfindung ist es vorgesehen, dass der Rohrreaktor zur Durchführung von Verfahrensschritt (b) mit Mischelementen zum Eintrag von zusätzlicher Mischenergie ausgestattet ist; auf diese Weise können besonders gute Reinigungsergebnisse erzielt werden, da durch die zusätzlichen Mischelemente eine noch weiter verbesserte, besonders innige Verteilung von Waschmedium einerseits und aufzureinigendem Roharomaten andererseits erreicht wird. Bei den Mischelementen handelt es sich um Bleche, insbesondere Prall- oder Umlenkbleche, Blenden, statische Mischer oder Stromteiler. Erfindungsgemäß ist es bevorzugt, wenn 1 bis 15, insbesondere 2 bis 15, bevorzugt 2 bis 10, besonders bevorzugt 2 bis 5, Mischelemente in dem Rohrreaktor vorliegen.

Gemäß dieser Ausführungsform ist es bevorzugt, wenn die durch die im Rohrreaktor vorgesehenen Mischelemente eine Mischenergie (d.h. eine volumenbezogene Mischenergie) insgesamt von 10 bis 1.000 Joule/Liter, bevorzugt 10 bis 500 Joule/Liter, besonders bevorzugt 20 bis 200 Joule/Liter, eingetragen wird. Mit anderen Worten wird gemäß dieser Ausführungsform bevorzugt eine Mischenergie (d.h. eine volumenbezogene Mischenergie) von 10 bis 1.000 Joule/Liter, bevorzugt 10 bis 500 Joule/Liter, besonders bevorzugt 20 bis 200 Joule/Liter, insgesamt eingetragen.

Im Rahmen der vorliegenden Erfindung ist es vorgesehen, dass die Mischelemente derart ausgebildet sind, dass der Druckabfall pro Mischelement 0,1 bar bis 3,0 bar, bevorzugt 0,3 bis 1,5 bar, besonders bevorzugt 0,3 bis 0,8 bar, beträgt.

Was die Verweilzeit der Emulsion von Waschmedium einerseits und Roharomaten andererseits im Rohrreaktor im Rahmen von Verfahrensschritt (b) anbelangt, so kann diese in weiten Bereichen variieren. Besonders bevorzugt ist es, wenn die Verweilzeit im Rohrreaktor 0,1 bis 120 Sekunden, bevorzugt 0,1 bis 60 Sekunden, besonders bevorzugt 1 bis 30 Sekunden, beträgt. Auf diese Weise werden besonders gute Waschergebnisse erzielt, da zum einen eine ausreichende Mindestverweilzeit, andererseits jedoch auch ein ökonomischer Durchsatz gewährleistet wird.

Im Rahmen der Aufreinigung sind auch das Masse- und Phasenverhältnis zwischen aufzureinigenden nitrierten Rohprodukten einerseits und Waschmedium andererseits von Bedeutung, welche jeweils in weiten Bereichen variieren können.

Besonders gute Ergebnisse werden erhalten, wenn das Masseverhältnis zwischen aufzureinigenden nitrierten Rohprodukten einerseits und Waschmedium (d. h. frisch zudosiertem Waschmedium) andererseits im Bereich von 200 : 1 bis 1 : 10, bevorzugt im Bereich von 100 : 1 bis 1 : 5, besonders bevorzugt im Bereich von 10 : 1 bis 1 : 2, eingestellt wird.

Gleichermaßen werden besonders gute Ergebnisse dann erhalten, wenn das Phasenverhältnis (d. h. insbesondere das Phasenverhältnis im Waschapparat) zwischen aufzureinigenden nitrierten Rohprodukten einerseits und Waschmedium andererseits im Bereich von 25 : 1 bis 1 : 5, insbesondere im Bereich von 10 : 1 bis 1 : 2, bevorzugt im Bereich von 5 : 1 bis 1 : 1, eingestellt wird. Die Einstellung des Phasenverhältnisses kann insbesondere durch eine Kreisführung des Waschmediums nach Phasentrennung erzielt werden. Dies gewährleistet einerseits eine optimale Austauschfläche zwischen Organphase und Waschmedium und andererseits eine möglichst kurze Zeit für die Phasentrennung im Phasentrennapparat.

Die Wäsche der Nitroaromaten wird üblicherweise als flüssig/flüssig-Wäsche (d. h. bei Temperaturen, bei denen der zu waschende bzw. aufzureinigende Nitroaromat - ebenso wie das Waschmedium - als Flüssigkeit vorliegt) durchgeführt.

Was das erfindungsgemäß eingesetzte Waschmedium anbelangt, so ist dieses unter Verfahrensbedingungen, insbesondere bei Temperaturen ab 5 °C, insbesondere bei Temperaturen ab 25 °C, und Atmosphärendruck, flüssig ausgebildet. Erfindungsgemäß bevorzugt wird ein wässrig basiertes Waschmedium, vorzugsweise Wasser, eingesetzt.

Je nach Phasenverhältnis im Waschapparat wird dabei der zu waschende Nitroaromat im Waschmedium als Öl-in-Wasser-Emulsion (O/W-Emulsion) oder das Waschmedium im zu waschenden Aromat als Wasser-in-Öl-Emulsion (W/O-Emulsion) dispergiert.

Die Effizienz des Waschmediums kann noch dadurch gesteigert werden, dass dem Waschmedium mindestens eine Base zugesetzt werden kann. Die Base kann insbesondere ausgewählt sein aus der Gruppe von anorganischen Hydroxiden, Carbonaten, Hydrogencarbonaten, Sulfiten, Hydrogensulfiten und Ammoniak sowie deren Mischungen oder Kombinationen, bevorzugt aus der Gruppe von Natronlauge, Kalilauge, Natriumcarbonat, Kaliumcarbonat, Natriumhydrogencarbonat, Kaliumhydrogencarbonat, Ammoniak, Ammoniumcarbonat, Natriumsulfit und Natriumhydrogensulfit sowie deren Mischungen oder Kombinationen.

Die bei einer alkalischen Wäsche eingesetzte Menge an Alkali sollte insbesondere derart hoch sein, dass nicht nur alle Säuren quantitativ zu ihren Salzen umgesetzt werden können, sondern es sollte insbesondere ein Überschuss an Base eingesetzt werden, damit der pH-Wert in der Waschlauge derart hoch ist, dass auch schwache Säuren, wie Mononitrophenole quantitativ ausgewaschen werden können.

Der Gehalt an Alkali kann insbesondere 0,01 mol/l bis 0,4 mol/l, bevorzugt 0,02 mol/l bis 0,2 mol/l, mindestens aber das Doppelte der für die Neutralisation aller Nitrophenole benötigten Menge, betragen.

Besonders gute Ergebnisse werden dann erhalten, wenn der Gehalt an Base im Waschmedium 0,01 bis 0,4 mol/l, bevorzugt 0,02 bis 0,2 mol/l, beträgt.

Insbesondere sollte der Gehalt an Base im Waschmedium mindestens das Doppelte der für die Neutralisation aller als Verunreinigungen enthaltener Nitrophenole theoretisch benötigten Alkalimenge betragen.

Wie zuvor ausgeführt, sollte also das Phasenverhältnis von zu waschendem Nitroaromat und frisch zudosiertem Waschmedium vorteilhafterweise 200 : 1 bis 1 : 10, bevorzugt 100 : 1 bis 1 : 5, besonders bevorzugt 10 : 1 bis 1 : 2, betragen. Durch eine Kreisführung des Waschmediums nach Phasentrennung kann dabei im Waschapparat ein Phasenverhältnis zwischen zu waschendem Nitroaromat und Waschmedium von 25 : 1 bis 1 : 5, insbesondere 10 : 1 bis 1 : 2, besonders bevorzugt 5 : 1 bis 1 : 1, eingestellt werden, um einerseits eine optimale Austauschfläche zwischen Organphase und Waschmedium zu erzeugen und andererseits die Zeit für die Phasentrennung im Phasentrennapparat so kurz wie möglich zu halten.

Je nach Phasenverhältnis im Waschapparat wird dabei der zu waschende Nitroaromat im Waschmedium als Öl-in-Wasser-Emulsion (O/W- Emulsion) oder das Waschmedium im zu waschenden Aromat als Wasser-in-Öl-Emulsion (W/O-Emulsion) dispergiert (vgl. obige Ausführungen).

Als Treibstrahl dient dabei, in Abhängigkeit vom gewählten Phasenverhältnis, entweder der zu waschende Aromat oder das Waschmedium, um den gewünschten Emulsionstyp einzustellen.

Die Fließgeschwindigkeit der Waschemulsion nach dem Strahlmischer im nachfolgenden Rohrreaktor kann insbesondere im Bereich von 0,1 bis 15,0 m/s, bevorzugt 0,5 bis 10 m/s, liegen.

Das Verhältnis der Geschwindigkeit zwischen zentralem Strahl und umgebenden Medium bewegt dabei - wie zuvor angeführt - sich zwischen 1 : 5 bis 30 : 1, bevorzugt 1 : 2 bis 20 : 1 und besonders bevorzugt zwischen 1 : 1 bis 10 : 1.

Um die Koaleszenz der Waschemulsion nach kurzer Zeit und damit eine nicht vollständige Extraktion der aus dem zu reinigenden Nitroaromaten zu entfernenden Verunreinigung zu verhindern, ist es vorteilhaft, durch zusätzlichen Eintrag von Mischenergie die Waschemulsion solange stabil zu halten, bis alle Verunreinigungen aus dem Nitroaromaten ausgewaschen sind und durch weitere Umsetzungen im Waschmedium an der Rückextraktion in den zu waschenden Nitroaromaten gehindert werden. Diese zusätzliche Mischenergie wird in das Gemisch der zwei nicht miteinander mischbaren Phasen durch eine Einspeisung in einen Reaktor mit zusätzlichen Mischeinrichtungen, bevorzugt in einen Rohrreaktor ohne Rückvermischung, eingebracht, wobei im Rohrreaktor durch zusätzliche Mischelemente, über den Rohrreaktor verteilt, nämlich Blenden, Umlenkbleche, Strömungsbrecher oder Statikmischer, die Emulsion vom Typ O/W bzw. W/O aufrechterhalten wird. Bevorzugterweise können 1 bis 15, insbesondere 2 bis 15, bevorzugt 2 bis 10 und besonders bevorzugt 2 bis 5 Mischelemente in dem Rohrreaktor vorliegen, wobei der Strahlmischer als Mischelement mitzählt.

Die insgesamt einzutragende volumenbezogene Mischenergie sollte 10 bis 1000 J/l, bevorzugt 10 bis 500 J/l und besonders bevorzugt 20 bis 200 J/l betragen.

Der Druckverlust pro Mischelement beträgt 0,1 bis 3,0 bar, bevorzugt 0,2 bis 1,5 bar und besonders bevorzugt 0,2 bis 0,8 bar, um die Anzahl der im Rohrreaktor benötigten zusätzlichen Mischelemente so niedrig wie möglich und die Verweilzeit in der Phasentrenneinrichtung so kurz wie möglich zu halten.

Die Verweilzeit in dem Rohrreaktor zur Abtrennung von Säuren, gefolgt von einer schnellen Weiterreaktion, wie z. B. einer Neutralisation, aus dem zu waschenden Nitroaromaten, wie Salpetersäure, Schwefelsäure, Mono-, Di- und Trinitrophenolen und Kresolen, Nitrobenzoesäuren etc., bei einer Wäsche mit Alkali, wie z. B. Natronlauge, Soda, Bicarbonat, Ammoniak, Kalilauge etc., sollte nicht mehr als 0,1 bis 120 Sekunden, bevorzugt 0,1 bis 60 Sekunden, besonders bevorzugt 1 bis 30 Sekunden, betragen.

Zur Entfernung von Verunreinigungen aus dem zu waschenden Nitroaromaten mit hohen Verteilungskoeffizienten zugunsten des zu waschenden Nitroaromaten, hohen Massentransferwiderständen in der Organphase und langsamen Weiterreaktionen der extrahierten Verunreinigung im Waschmedium, wie z.B. Nitrose oder Stickstoffdioxid, sollte die Verweilzeit im nachfolgenden Reaktor an diese Verhältnisse angepasst werden (wie z. B. durch eine Kombination der vorstehend beschriebenen Einrichtungen zur Erzeugung einer optimalen Waschemulsion mit Rührkesseln, um die notwendige Verweilzeit zu erzeugen). Gemäß einer besonderen Ausführungsform des erfindungsgemäßen Verfahrens wird dies insbesondere erreicht durch eine Kombination der vorstehend beschriebenen Einrichtungen zur Erzeugung einer optimalen Waschemulsion mit Rührkesseln, um die notwendige Verweilzeit für den Phasentransfer und die nachfolgende Umsetzung sicherzustellen.

Wie zuvor bereits ausgeführt, sollte die bei einer alkalischen Wäsche eingesetzte Menge an Alkali derart hoch sein, dass nicht nur alle Säuren quantitativ zu ihren Salzen umgesetzt werden können, sondern es sollte ein Überschuss an Base eingesetzt werden, damit der pH-Wert in der Waschlauge derart hoch ist, dass auch schwache Säuren, wie Mononitrophenole quantitativ ausgewaschen werden können. Wie zuvor angeführt, sollte dabei der Gehalt an Alkali insbesondere 0,01 mol/l bis 0,4 mol/l, bevorzugt 0,02 mol/l bis 0,2 mol/l, mindestens aber das Doppelte der für die Neutralisation aller Nitrophenole benötigten Menge, betragen.

Die am Ende der Mischstrecke vorliegende Emulsion kann beispielsweise in einem Phasentrennapparat (z. B. Scheider bzw. Settler) wieder in die einzelnen Phasen getrennt werden. Das Waschmedium mit den darin enthaltenen Verunreinigungen kann entweder als Abwasser einer Abwasserbehandlung zugeführt oder im Gegenstrom in die vorgeschaltete Waschstufe eingebracht werden.

Der gewaschene Nitroaromat kann entweder in die nachfolgende Waschstufe eingespeist oder am Ende der Wäsche direkt zur Weiterverarbeitung oder in ein Zwischenlager transferiert werden.

Als Phasentrennapparat können alle Arten von statischen Scheidern zum Einsatz kommen, aber auch dynamische Scheider, wie Zentrifugalseparatoren. Die Scheidezeit der Emulsion Nitroaromat/Waschmedium hängt außer vom Emulsionstyp (W/O oder O/W) und der eingetragenen Mischenergie zusätzlich auch von dem Überschuss an Base im Waschmedium, der nicht zur Neutralisation benötigt wird, ab. Bei Eintrag gleicher Mischenergie sinkt die Scheidezeit deutlich mit zunehmender Basekonzentration im Waschmedium. Zur Beschleunigung der Phasentrennung können aber auch oberflächenaktive Mittel oder auch mechanische Trennhilfen, wie Packungen, Trennbleche etc., eingesetzt werden. Auch durch einen auf den Nitroaromaten und Emulsionstyp abgestimmten Abstand zwischen den einzelnen Mischelementen kann die Phasentrennung beschleunigt werden.

Was die aufzureinigenden nitrierten Rohprodukte anbelangt, so sind diese im Allgemeinen unter Verfahrensbedingungen, insbesondere bei Temperaturen ab 5 °C, insbesondere bei Temperaturen ab 25 °C, und Atmosphärendruck, flüssig ausgebildet. Insbesondere stammen die aufzureinigenden nitrierten Rohprodukte aus der Nitrierung von ein- oder mehrkernigen Aromaten, insbesondere aus der Nitrierung von Benzol, Toluol, Xylol oder halogenierten Aromaten, wie insbesondere chlorierten Benzolen.

Bei den aufzureinigenden nitrierten Rohprodukten handelt es sich insbesondere um gegebenenfalls halogenierte Mono-, Di- und Trinitroaromaten, wie z. B. Nitrobenzol (MNB), Mononitrotoluol (MNT), Dinitrotoluol (DNT), Trinitrotoluol (TNT), Nitrochlorbenzol (MNCB) oder dergleichen.

Im Allgemeinen schließt sich dem Verfahrensschritt (b) eine Abtrennung der von den Verunreinigungen befreiten nitrierten Produkte vom Waschmedium an. Diese Abtrennung erfolgt im Allgemeinen mittels einer geeigneten Scheideeinrichtung (Scheider bzw. Settler).

Weiterhin kann es gemäß einer besonderen Ausführungsform des erfindungsgemäßen Verfahrens vorgesehen sein, dass das aus dem Rohrreaktor austretende Gemisch aus gereinigten nitrierten Produkten und Waschmedium, insbesondere noch vor Abtrennung der von den Verunreinigungen befreiten nitrierten Produkte vom Waschmedium, zunächst in einen Rührkessel überführt wird. Auf diese Weise wird die Kontakt- und/oder Verweilzeit zwischen aufzureinigenden Nitrierprodukten einerseits und Waschmedium andererseits in effizienter Weise verlängert, so dass gegebenenfalls noch nicht ausgewaschene Verunreinigungen in das Waschmedium überführt bzw. hierdurch neutralisiert werden.

Gemäß einer vorteilhaften Ausführungsform des erfindungsgemäßen Verfahrens ist es vorgesehen, dass das Waschmedium, insbesondere nach Abtrennung der von den Verunreinigungen befreiten nitrierten Produkte vom Waschmedium, rezykliert wird. Auf diese Weise wird eine effiziente Wäsche bzw. Kreisführung ermöglicht und die Menge an Waschmedium auf ein Minimum reduziert.

Gegebenenfalls können nach der Wäsche bzw. nach Abtrennung des Waschmediums (z. B. nach einer Trennung der Waschemulsion in einem statischen Scheider oder durch einen Zentrifugalseparator) gegebenenfalls noch vorhandene Restmengen bzw. Spuren an Wasser, insbesondere suspendiertes und/oder gelöstes Wasser, durch Trocknung aus dem aufgereinigten Nitroaromaten entfernt werden.

Das erfindungsgemäße Verfahren eignet sich zur Durchführung der sauren Wäsche und/oder der basischen Wäsche und/oder der neutralen Wäsche der nitrierten Rohprodukte. Das erfindungsgemäße Verfahren kann also in allen drei vorgenannten Waschschritten zum Einsatz kommen. Gleichermaßen ist es aber auch möglich, das erfindungsgemäße Verfahren nur für eine oder zwei Waschstufen zu verwenden, beispielsweise nur für die saure Wäsche oder aber nur für die basische Wäsche oder aber nur für die neutrale Wäsche. In dieser Hinsicht ist das erfindungsgemäße Verfahren flexibel einsetzbar.

Wie zuvor geschildert, ist das erfindungsgemäße Verfahren mit einer Vielzahl von Vorteilen und Besonderheiten verbunden, von denen nachfolgend einige Vorteile und Besonderheiten - jedoch nicht abschließend und in nicht beschränkender Weise - aufgeführt werden sollen:

Insbesondere ermöglicht das erfindungsgemäße Verfahren eine effiziente Aufreinigung von aus bei der Nitrierung von nitrierbaren aromatischen Verbindungen nach Abtrennung der Nitrierendsäure anfallenden nitrierten Rohprodukten mit nur geringer Komplexität und großer Verfahrensökonomie wie Verfahrenseffizienz.

Der erfindungsgemäß eingesetzte Rohrreaktor ermöglicht eine effiziente und innige Verteilung von Waschmedium einerseits und nitrierten Roharomaten andererseits ineinander, so dass keine weiteren Waschschritte oder anderweitige Behandlungsschritte erforderlich sind. Die Wasch- bzw. Behandlungseffizienz wird noch dadurch gesteigert, dass in dem Rohrreaktor zusätzliche Mischelemente vorgesehen sind, wie zuvor geschildert, welche die Durchmischung noch weiterführend verbessern.

Der erfindungsgemäß für die Aufreinigung zum Einsatz kommende Rohrreaktor kann gleichermaßen bei der vorangehenden Nitrierung als Reaktionsbehältnis eingesetzt werden, so dass keine zusätzliche Apparatur für die Aufreinigung der nitrierten Rohprodukte eingesetzt zu werden braucht.

Der erfindungsgemäß für die Aufreinigung der rohen Nitrierprodukte zum Einsatz kommende Rohrreaktor ermöglicht die Erzeugung großer Austauschflächen für ein zweiphasiges Gemisch aus Waschmedium einerseits und nitrierten Roharomaten andererseits, so dass auf diese Weise ein effektiver Massentransfer und eine schnelle Überführung der Verunreinigungen in das Waschmedium bzw. im Fall saurer Verbindungen eine schnelle Neutralisation gewährleistet wird.

Weiterhin ermöglicht die erfindungsgemäße Verfahrensführung eine schnelle und gleichsam effiziente Beseitigung der aus der Nitrierung stammenden Verunreinigungen aus den nitrierten Rohprodukten, wobei das Waschmedium nach der Behandlung der nitrierten Roharomaten ohne Weiteres rezykliert bzw. im Kreis gefahren werden kann.

Zur Durchführung des zuvor geschilderten Verfahrens eignet sich eine Vorrichtung (Anlage) zur Entfernung von Verunreinigungen aus bei der Nitrierung von nitrierbaren aromatischen Verbindungen nach Abtrennung der Nitrierendsäure anfallenden nitrierten Rohprodukten durch Behandlung mit einem Waschmedium,
wobei die Vorrichtung die folgenden Einrichtungen aufweist:
(a) mindestens eine Dispergiereinrichtung, insbesondere mindestens ein Mischorgan, zum Inkontaktbringen und Emulgieren von aufzureinigenden nitrierten Rohprodukten einerseits und Waschmedium andererseits; und,
(b) stromabwärts zur Dispergiereinrichtung angeordnet, einen Rohrreaktor zur Einspeisung der in der Dispergiereinrichtung erzeugten Emulsion von aufzureinigenden nitrierten Rohprodukten einerseits und Waschmedium andererseits, wobei der Rohrreaktor derart ausgebildet ist, dass während des Durchtritts der Emulsion durch den Rohrreaktor eine Entfernung der in den nitrierten Rohprodukten anfänglich vorhandenen Verunreinigungen ermöglicht wird und/oder dass während des Durchtritts der Emulsion durch den Rohrreaktor die in den nitrierten Rohprodukten anfänglich vorhandenen Verunreinigungen in das Waschmedium überführt und/oder hierdurch neutralisiert werden, wobei der Rohrreaktor mit Mischelementen zum Eintrag von zusätzlicher Mischenergie ausgestattet ist, wobei die Mischelemente als Bleche, als Blenden, als statische Mischer oder als Stromteiler ausgebildet sind, wobei der Druckabfall pro Mischelement 0,1 bar bis 3,0 bar beträgt.

Wie zuvor im Zusammenhang mit dem erfindungsgemäßen Verfahren geschildert, kann die Dispergiereinrichtung, insbesondere das Mischorgan, ein Rührkessel, ein Strahlmischer (Jet-Mixer) oder eine Pumpe, insbesondere eine Kreiselpumpe, vorzugsweise eine Pumpe, insbesondere eine Kreiselpumpe, oder ein Strahlmischer (Jet-Mixer), besonders bevorzugt ein Strahlmischer (Jet-Mixer), sein.

Wie zuvor im Rahmen des erfindungsgemäßen Verfahrens beschrieben, kann die Dispergiereinrichtung, insbesondere das Mischorgan, dem Reaktor vorgeschaltet, insbesondere unmittelbar vorgeschaltet, sein. Insbesondere kann es in diesem Zusammenhang vorgesehen sein, dass die Dispergiereinrichung, insbesondere das Mischorgan, in den Rohrreaktor übergeht.

Gemäß einer alternativen Ausführungsform kann die Dispergiereinrichtung, insbesondere das Mischorgan, in den Rohrreaktor integriert sein und/oder Bestandteil des Rohrreaktors sein. Diesbezüglich kann auf die obigen Ausführungen im Zusammenhang mit dem erfindungsgemäßen Verfahren verwiesen werden.

Wie zuvor bei der Schilderung des erfindungsgemäßen Verfahrens erläutert, ist der Rohrreaktor mit Mischelementen zum Eintrag von zusätzlicher Mischenergie ausgestattet. Wie zuvor beschrieben, sind die Mischelemente als Bleche, insbesondere Prall- oder Umlenkbleche, als Blenden, als statische Mischer oder als Stromteiler ausgebildet.

Im Rahmen der erfindungsgemäß eingesetzten Vorrichtung kann eine ein-, zwei- oder dreistufige Wäsche des rohen Nitrierproduktes durchgeführt werden (d. h. saure Wäsche und/oder basische Wäsche und/oder neutrale Wäsche).

Weiterhin kann es vorgesehen sein, dass - stromabwärts zum Rohrreaktor angeordnet - eine Abtrenneinrichtung, insbesondere eine Scheideeinrichtung (Scheider bzw. Settler und/oder dynamischer Scheider bzw. Zentrifugalseparator), zur Abtrennung der von den Verunreinigungen befreiten nitrierten Produkte vom Waschmedium angeordnet ist.

Des Weiteren besteht im Rahmen der erfindungsgemäß eingesetzten Vorrichtung die Möglichkeit, dass - stromabwärts zum Rohrreaktor und stromaufwärts zur Abtrenneinrichtung (d. h. mit anderen Worten zwischen Rohrreaktor und Abtrenneinrichtung) - ein Rührkessel und/oder Rührreaktor angeordnet ist. Insbesondere wird auf diese Weise die Kontakt- und/oder Verweilzeit zwischen nitrierten Produkten einerseits und dem Waschmedium andererseits verlängert.

Für weitergehende Einzelheiten zu der erfindungsgemäß eingesetzten Vorrichtung bzw. Anlage kann auf die obigen Ausführungen zu dem erfindungsgemäßen Verfahren verwiesen werden, welche in Bezug auf die erfindungsgemäß eingesetzte Vorrichtung bzw. Anlage entsprechend gelten.

Schließlich ist weiterer Gegenstand der vorliegenden Erfindung - gemäß einem **zweiten** Aspekt der vorliegenden Erfindung - eine Produktionsanlage zur Nitrierung nitrierbarer aromatischer Verbindungen mit nachfolgender Aufreinigung der bei der Nitrierung entstehenden nitrierten Rohprodukte,
**dadurch gekennzeichnet**,
dass die Produktionsanlage die folgenden Einheiten umfasst:
(a) eine Nitriereinheit zur Nitrierung aromatischer Verbindungen, insbesondere mit einem oder mehreren entsprechenden Reaktionsbehältern zur Durchführung der Nitrierreaktion(en);
(b) gegebenenfalls, in Produktionslinie stromabwärts zur Nitriereinheit angeordnet, mindestens eine Abtrenneinrichtung, insbesondere eine Scheideeinrichtung (Scheider), zur Abtrennung der Nitrierendsäure von den nitrierten Rohprodukten;
(c) in Produktionslinie stromabwärts zur Nitriereinheit und zur gegebenenfalls vorhandenen Abtrenneinrichtung angeordnet, mindestens eine Wascheinrichtung zur Durchführung einer Wäsche der nitrierten Rohprodukte, wobei die Wascheinrichtung umfasst:
   - mindestens eine Dispergiereinrichtung, insbesondere mindestens ein Mischorgan, zum Inkontaktbringen und Emulgieren der aufzureinigenden nitrierten Rohprodukte einerseits und dem Waschmedium andererseits und,
   - stromabwärts zur Dispergiereinrichtung angeordnet, einen Rohrreaktor zur Einspeisung der in der Dispergiereinrichtung erzeugten Emulsion von aufzureinigenden nitrierten Rohprodukten einerseits und Waschmedium andererseits, wobei der Rohrreaktor derart ausgebildet ist, dass während des Durchtritts der Emulsion durch den Rohrreaktor eine Entfernung der in den nitrierten Rohprodukten anfänglich vorhandenen Verunreinigungen ermöglicht wird und/oder dass während des Durchtritts der Emulsion durch den Rohrreaktor die in den nitrierten Rohprodukten anfänglich vorhandenen Verunreinigungen in das Waschmedium überführt und/oder hierdurch neutralisiert werden, wobei der Rohrreaktor mit Mischelementen zum Eintrag von zusätzlicher Mischenergie ausgestattet ist, wobei die Mischelemente als Bleche, als Blenden, als statische Mischer oder als Stromteiler ausgebildet sind, wobei der Druckabfall pro Mischelement 0,1 bar bis 3,0 bar beträgt;
(d) gegebenenfalls, in Produktionslinie stromabwärts zur Wascheinrichtung angeordnet, einen Rührkessel, insbesondere zur Erhöhung der Kontakt- und/oder Verweilzeit zwischen nitrierten Produkten einerseits und Waschmedium andererseits;
(e) in Produktionslinie stromabwärts zur Wascheinheit und zum gegebenenfalls vorhandenen Rührkessel angeordnet, eine Abtrenneinrichtung, insbesondere eine Scheideeinrichtung (Scheider), zur Abtrennung der von den Verunreinigungen befreiten nitrierten Produkte vom Waschmedium.

Mit anderen Worten ist bei der erfindungsgemäßen Produktionsanlage die zuvor beschriebene Vorrichtung bzw. Anlage zur Aufreinigung, d. h. zur Entfernung von Verunreinigungen, Bestandteil dieser Produktionsanlage, nämlich in Form der Wascheinheit bzw. Wascheinrichtung (c).

Wie zuvor geschildert, kann auch bei der erfmdungsgemäßen Produktionsanlage die Dispergiereinrichtung, insbesondere das Mischorgan, ein Rührkessel, ein Strahlmischer (Jet-Mixer) oder eine Pumpe, insbesondere eine Kreiselpumpe, vorzugsweise eine Pumpe, insbesondere eine Kreiselpumpe, oder ein Strahlmischer (Jet-Mixer), besonders bevorzugt ein Strahlmischer (Jet-Mixer), sein.

Gemäß einer besonderen Ausgestaltung der erfindungsgemäßen Produktionsanlage kann - wie zuvor geschildert - die Dispergiereinrichtung, insbesondere das Mischorgan, dem Reaktor vorgeschaltet, insbesondere unmittelbar vorgeschaltet, sein. Insbesondere kann bei dieser Ausführungsform die Dispergiereinrichung, insbesondere das Mischorgan, in den Rohrreaktor übergehen.

Gleichermaßen kann es erfindungsgemäß vorgesehen sein, dass die Dispergiereinrichtung, insbesondere das Mischorgan, in den Rohrreaktor integriert ist und/oder Bestandteil des Rohrreaktors ist. Bezüglich dieser Ausführungsform kann - zur Vermeidung unnötiger Wiederholungen - auf die obigen Ausführungen verwiesen werden.

Wie zuvor im Zusammenhang mit dem erfindungsgemäßen Verfahren und im Zusammenhang mit der erfindungsgemäß eingesetzten Vorrichtung bzw. Anlage zur Aufreinigung geschildert, ist der Rohrreaktor mit Mischelementen zum Eintrag von zusätzlicher Mischenergie ausgestattet. Dabei sind die Mischelemente als Bleche, insbesondere Prall- oder Umlenkbleche, als Blenden, als statische Mischer oder als Stromteiler ausgebildet.

Das erfindungsgemäße Verfahren eignet sich insbesondere zur Durchführung einer sauren Wäsche und/oder einer basischen Wäsche und/oder einer neutralen Wäsche von nitrierten Rohprodukte. Das erfindungsgemäße Verfahren kann also in allen drei vorgenannten Waschschritten einer Wascheinrichtung zum Einsatz kommen. Gleichermaßen ist es aber auch möglich, das erfindungsgemäße Verfahren nur für eine oder zwei Waschschritte zu verwenden, beispielsweise nur für eine saure Wäsche oder aber nur für eine basische Wäsche oder aber nur für eine neutrale Wäsche. In dieser Hinsicht ist das erfindungsgemäße Verfahren flexibel einsetzbar.

Für weitergehende Einzelheiten zu der erfindungsgemäßen Produktionsanlage kann auf die obigen Ausführungen zu dem erfindungsgemäßen Verfahren und zu der erfindungsgemäß eingesetzten Vorrichtung bzw. Anlage verwiesen werden, welche in Bezug auf die erfindungsgemäße Produktionsanlage entsprechend gelten.

Das erfindungsgemäße Verfahren und die erfindungsgemäß eingesetzte Vorrichtung bzw. Anlage zur Aufreinigung sowie die erfindungsgemäße Produktionsanlage zur Nitrierung sind in den beigefügten Figurendarstellungen beispielhaft und in nichtbeschränkender Weise veranschaulicht.

Weitere Vorteile, Eigenschaften, Aspekte und Merkmale der vorliegenden Erfindung ergeben sich aus der folgenden Beschreibung von in den Zeichnungen dargestellten, erfindungsgemäß bevorzugten Ausführungsformen. Es zeigt:
- Fig. 1: eine schematische Darstellung einer Wäsche von Nitroaromaten nach dem Stand der Technik mittels Mixer/Settler-Technologie für die üblichen drei Waschstufen einer Wäsche von Nitroaromaten;
- Fig. 2: eine schematische Darstellung einer einstufigen Wäsche für Nitroaromaten nach dem erfindungsgemäßen Verfahren bzw. mit der erfindungsgemäß eingesetzten Vorrichtung bzw. Anlage;
- Fig. 3: eine schematische Darstellung eines Ablaufs des erfindungsgemäßen Verfahrens bzw. eine schematische Darstellung der erfindungsgemäß eingesetzten Vorrichtung bzw. Anlage gemäß einem bevorzugten Ausführungsbeispiel der Erfindung für die üblichen drei Waschstufen einer Wäsche von Nitroaromaten;
- Fig. 4: eine schematische Darstellung einer erfindungsgemäßen Produktionsanlage zur Nitrierung nitrierbarer aromatischer Verbindungen mit nachfolgender Wäsche der erhaltenen Nitroaromaten gemäß einem bevorzugten Ausführungsbeispiel der Erfindung.

Fig. 1 zeigt ein Beispiel für eine Wäsche von Nitroaromaten in drei Schritten nach dem Stand der Technik:
a) In Schritt 1 werden in einer mehrstufigen kontinuierlichen sauren Wäsche (WS) die im rohen Nitroaromaten (NA 10) suspendierte und gelöste Schwefel- und Salpetersäure durch Wäsche mit Frischwasser (WW 10) ausgewaschen. Der zu waschende Nitroaromat (NA 10) und das Waschwasser (WW 10) werden in eine Mischeinrichtung, üblicherweise einem Rührkessel, mit einer Verweilzeit von ca. 10 Minuten. eingespeist. Die gebildete Waschemulsion wird anschließend in einem Scheider (S) getrennt. Zur vollständigen Entfernung der gelösten und suspendierten Mineralsäuren können bis zu 4 Mixer/Settler-Einheiten (n = 3) zum Einsatz kommen, wobei das Waschmedium und der zu waschende Nitroaromat im Gegenstrom geführt werden. Das Waschmedium wird, nach Phasentrennung, entweder als Abwasser (WW 11) sofort vollständig abgegeben, oder eine Teilmenge wird zusätzlich im Kreis geführt, um ein vorgegebenes Phasenverhältnis und damit einen definierten Emulsionstyp einzustellen und um die Zeit zur Trennung der Phasen zu minimieren. Der von Mineralsäuren befreite Nitroaromat (NA 11) wird in Waschstufe 2, die alkalische Wäsche (WA), eingespeist.
b) In Schritt 2 werden in einer mehrstufigen kontinuierlichen alkalischen Wäsche (WA) alle gelösten Nitrophenole, Nitrobenzoesäuren und andere aciden Stoffe aus dem oxidativen Abbau von Verunreinigungen und isomere Nitroaromaten aus dem Nitroaromaten (z. B. TNT) entfernt. Der zu waschende Nitroaromat (NA 11) und das Waschwasser (WW 10 bzw. WW13) werden, zusammen mit einer Base, in einen Rührkessel mit einer Verweilzeit von ca. 10 Minuten. eingespeist. Die gebildete Waschemulsion wird anschließend in einem Scheider (S) getrennt. Zur vollständigen Entfernung der im Nitroaromat gelösten Nitrophenole, Nitrobenzoesäuren und anderen aciden Stoffen aus dem oxidativen Abbau von Verunreinigungen und isomeren Nitroaromaten können bis zu 4 Mixer/Settler-Einheiten (n = 3) zum Einsatz kommen, wobei das Waschmedium und der zu waschende Nitroaromat im Gegenstrom geführt werden. Das Waschmedium wird, nach Phasentrennung, entweder als Abwasser (WW 12) vollständig sofort abgegeben, oder eine Teilmenge wird zusätzlich noch im Kreis geführt, um ein vorgegebenes Phasenverhältnis und damit einen definierten Emulsionstyp einzustellen und um die Zeit zu Trennung der Phasen zu minimieren. Der von Mineralsäuren, Nitrophenolen, Nitrobenzoesäuren und anderen aciden Stoffe aus dem oxidativen Abbau von Verunreinigungen und isomeren Nitroaromaten befreite Nitroaromat (NA 12) wird in Waschstufe 3, die Neutralwäsche (WN), eingespeist.
c) In Schritt 3 werden in einer mehrstufigen Neutralwäsche (WN) die mitgerissenen Spuren an Waschmedium aus der alkalischen Wäsche (WA) entfernt. Der zu waschende Nitroaromat (NA 12) und das Waschwasser (WW 10) werden in einen Rührkessel mit einer Verweilzeit von ca. 10 Minuten. eingespeist. Die gebildete Waschemulsion wird anschließend in einem Scheider (S) getrennt. Zur vollständigen Entfernung der im Nitroaromat noch suspendierten bzw. gelösten Spuren an Base können bis zu 4 Mixer/Settler-Einheiten (n = 3) zum Einsatz kommen, wobei das Waschmedium und der zu waschende Nitroaromat im Gegenstrom geführt werden. Die wässrige Phase wird entweder als Waschmedium (WW 13) sofort vollständig in die alkalische Wäsche (WA) eingespeist, oder eine Teilmenge wird zusätzlich im Kreis geführt, um ein vorgegebenes Phasenverhältnis und damit einen definierten Emulsionstyp einzustellen und um die Zeit zu Trennung der Phasen zu minimieren.

Der jetzt von Mineralsäuren, Nitrophenolen, Nitrobenzoesäuren und anderen aciden Stoffe aus dem oxidativen Abbau von Verunreinigungen, isomeren Nitroaromaten und Restspuren Alkali befreite Nitroaromat (NA 13) wird direkt zur Weiterverarbeitung oder in ein Zwischenlager abgegeben.

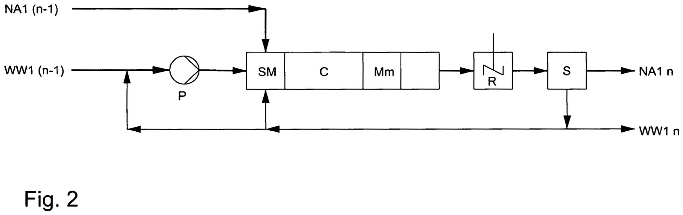

Fig. 2 zeigt eine Ausführungsform für eine Waschstufe nach dem erfindungsgemäßen Verfahren bzw. nach der erfindungsgemäß eingesetzten Vorrichtung bzw. Anlage zur Wäsche von Nitroaromaten mit dem Waschmedium als Treibstrahl.

Der zu waschende Nitroaromat, nach der Abtrennung der Nitrierendsäure (NA1 (n-1) mit n = 1)) oder nach der Entfernung der im Nitroaromaten als Mikroemulsion noch suspendierten Nitrierendsäure oder der im Nitroaromaten noch gelösten Schwefelsäure, Salpetersäure und Nitrose in einer sauren Wäsche (WS mit n = 2) oder nach der Entfernung aller im Nitroaromaten gelösten Nitrophenole, Nitrobenzoesäuren und anderen aciden Stoffen aus dem oxidativen Abbau von Verunreinigungen und isomerer Nitroaromaten aus dem Nitroaromaten (z.B. TNT) in Gegenwart von Basen in einer alkalische Wäsche (WA mit n = 3) wird in einem Strahlmischer (SM) mit dem Waschmedium WW1 (n-1), das im dargestellten Fall als Treibstrahl dient, zusammengebracht und direkt in einen Rohrreaktor (C) der zusätzliche Mischelementen (Mm) enthält, eingebracht.

Zur Einstellung einer verlängerten Verweilzeit, um langsam ablaufende Umsetzungen der auszuwaschenden Verunreinigungen im Waschmedium, wie Nitrose, zuzulassen, kann die Waschemulsion aus dem Rohrreaktor in einen Verweilzeitbehälter, wie z. B. einen oder mehrere Rührkessel (R), eingespeist werden. Die Waschemulsion aus dem Rohrreaktor wird direkt oder nach einer verlängerten Verweilzeit im Rührkessel in einer Scheideeinrichtung in die Phasen getrennt.

Der gewaschene Nitroaromat (NA1 n mit n = 1 bis 3) wird entweder in die nachfolgende Waschstufe bzw. als fertig gewaschenes Produkt (NA13) zur Weiterverarbeitung abgegeben. Das beladene Waschmedium (WW1 n mit n = 1 bis 3) wird entweder direkt als Abwasser abgegeben oder als Teilstrom zur Einstellung eines definierten Phasenverhältnisses zwischen Nitroaromat und Waschmedium zurückgeführt. Dieser zurückgeführte Teilstrom kann entweder zusammen mit dem neu zugesetzten Waschwasser als Treibstrahl oder als Umlaufstrom direkt in den Rohrreaktor eingespeist werden.

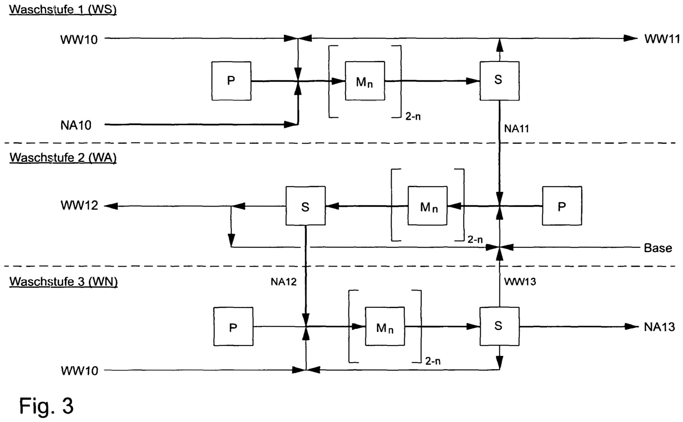

Fig. 3 zeigt ein Beispiel des erfindungsgemäßen Verfahrens in drei Schritten für die separate Entfernung der Mineralsäuren mittels saurer Wäsche (WS), die Entfernung aller gelöster Nitrophenole, Nitrobenzoesäuren und anderer acider Stoffe aus dem oxidativen Abbau von Verunreinigungen und isomerer Nitroaromaten in Gegenwart von Basen im alkalischen Bereich mittels alkalischer Wäsche (WA) und eine Neutralwäsche (WN).
a) In Schritt 1 werden in einer einstufigen sauren Wäsche (WS) die im rohen Nitroaromaten (NA 10) suspendierte und gelöste Schwefel- und Salpetersäure durch Wäsche mit Frischwasser (WW 10) entfernt. Der zu waschende Nitroaromat (NA 10) und das Waschwasser (WW 10) werden mittels Pumpen (P) über einen Strahlmischer oder direkt in einen Rohrreaktor eingespeist, der zusätzliche Mischelemente (Mn) enthält. Nach Durchlauf durch den Rohrreaktor wird die gebildete Emulsion in einem Scheider (S) getrennt. Das Waschmedium wird, nach Phasentrennung, entweder als Abwasser (WW 11) direkt abgegeben, oder eine Teilmenge wird zusätzlich im Kreis geführt für die Einstellung eines vorgegebenen Phasenverhältnisses und damit eines definierten Emulsionstyps und um die Zeit zur Trennung der Phasen zu minimieren. Der von Mineralsäuren befreite Nitroaromat (NA 11) wird in Waschstufe 2, die alkalische Wäsche (WA), eingespeist.
b) In Schritt 2 werden in einer einstufigen alkalischen Wäsche (WA) alle gelösten Nitrophenole, Nitrobenzoesäuren und anderen aciden Stoffe aus dem oxidativen Abbau von Verunreinigungen und isomeren Nitroaromaten aus dem Nitroaromaten entfernt. Der zu waschende Nitroaromat (NA 11) nach der sauren Wäsche (WS) und das Waschwasser (WW 10 bzw. WW 13 aus der Neutralwäsche) und eine Base werden mittels Pumpen (P) über einen Strahlmischer oder direkt in einen Rohrreaktor eingespeist, der zusätzliche Mischelemente (Mn) enthält. Nach Durchlauf durch den Rohrreaktor wird die gebildete Emulsion in einem Scheider getrennt. Das Waschmedium, das alle gelösten Nitrophenole, Nitrobenzoesäuren und andere acide Stoffe aus dem oxidativen Abbau von Verunreinigungen und extrahierte isomere Nitroaromaten (als Salz gelöst) enthält, wird nach Phasentrennung entweder als Abwasser (WW 12) direkt abgegeben, oder eine Teilmenge wird im Kreis geführt zur Einstellung eines vorgegebenen Phasenverhältnisses und damit eines definierten Emulsionstyps und um die Zeit zur Trennung der Phasen zu minimieren. Der von Mineralsäuren, Nitrophenolen, Nitrobenzoesäuren und andere aciden Stoffen aus dem oxidativen Abbau von Verunreinigungen und isomeren Nitroaromaten befreite Nitroaromat (NA 12) wird in Waschstufe 3, die Neutralwäsche (WN), eingespeist.
c) In Schritt 3 werden in einer einstufigen Neutralwäsche (WN) die mitgerissenen Spuren an Waschmedium aus der alkalischen Wäsche entfernt. Der zu waschende Nitroaromat (NA 12) und das Waschwasser (WW 10) werden mittels Pumpen (P) über einen Strahlmischer oder direkt in einen Rohrreaktor eingespeist, der zusätzliche Mischelemente (Mn) enthält. Nach Durchlauf durch den Rohrreaktor wird die gebildete Emulsion in einem Scheider (S) getrennt. Das Waschmedium, das die Restspuren an Alkali und Verunreinigungen enthält, wird entweder als Abwasser (WW 13) direkt in die Waschstufe 2 (WA) eingeführt, oder eine Teilmenge wird zusätzlich im Kreis geführt zur Einstellung eines vorgegebenen Phasenverhältnisses und damit eines definierten Emulsionstyps und um die Zeit zur Trennung der Phasen zu minimieren. Der jetzt von Mineralsäuren, Nitrophenolen, Nitrobenzoesäuren und andere aciden Stoffen aus dem oxidativen Abbau von Verunreinigungen, isomeren Nitroaromaten und Restspuren Alkali befreite Nitroaromat (NA 13) wird direkt zur Weiterverarbeitung oder in ein Zwischenlager abgegeben.

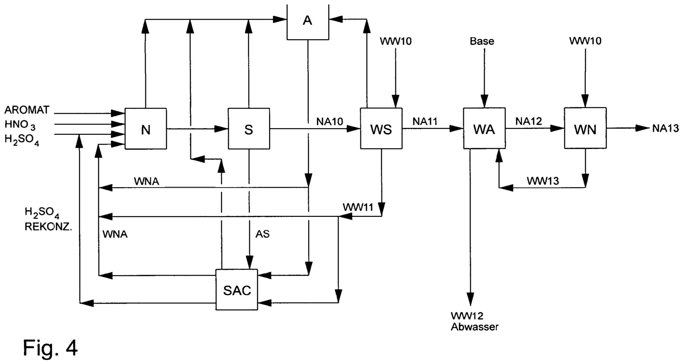

Fig. 4 zeigt ein Beispiel für eine Produktionsanlage zur Herstellung von Nitroaromaten mit integrierter erfindungsgemäßer Wäsche der rohen Nitroaromaten aus einer isothermen oder adiabatischen Nitrierung. Der in der Nitriereinheit (N) durch Umsetzung des Aromaten mit Salpetersäure in Gegenwart von Schwefelsäure gebildete rohe Nitroaromat (NA 10) wird nach Abtrennung der Nitriersäure im Scheider (S) in der sauren Wäsche (WS) mit Wasser (WW 10) in der erfindungsgemäßen Weise gewaschen. Nach Phasentrennung wird das resultierende Abwasser (WW 11), das alle ausgewaschene Schwefel- und Salpetersäure enthält, zusammen mit der aus der Abgasbehandlung der Nitrieranlage in einer Absorberanlage (A) erhaltenen Salpetersäure (WNA), entweder direkt oder nach Aufkonzentrierung in einer SAC-Anlage (SAC), zusammen mit der Endsäure (AS) aus der Nitrierung wieder in die Nitrierung zurückgeführt oder als zu behandelndes Abwasser abgegeben.

Der von den Mineralsäuren befreite Nitroaromat (NA 11) wird in der Waschstufe 2 (alkalische Wäsche WA) in Gegenwart von Basen nach dem erfindungsgemäßen Verfahren quasi einstufig gewaschen. Nach Phasentrennung wird das Abwasser aus der alkalischen Wäsche (WW12) mit einem pH-Wert im Bereich von 8,0 bis 13, das alle Nitrophenole, Nitrobenzoesäuren und anderen aciden Stoffe aus dem oxidativen Abbau von Verunreinigungen und isomeren Nitroaromaten (z. B. TNT) enthält, vor Abgabe in einen Vorfluter einer zusätzlichen Behandlung zugeführt, wie z. B. einer Thermolyse.

Der Nitroaromat (NA 12) aus der alkalischen Wäsche (WA) wird in die Neutralwäsche (WN) eingespeist und mit Wasser (WW 10) nach dem erfindungsgemäßen Verfahren quasi einstufig gewaschen. Nach Phasentrennung wird das Abwasser (WW 13) aus der Neutralwäsche (WN) in die Waschstufe 2 (WA) zusammen mit Base eingespeist. Der gewaschene Nitroaromat (NA 13) wird in die Weiterverarbeitung, wie z. B. in eine Isomerentrennung, die Reduktion zu dem entsprechenden Amin oder in ein Zwischenlager, abgegeben.

Weitere Ausgestaltungen, Abwandlungen, Variationen der vorliegenden Erfindung sind für den Fachmann beim Lesen der Beschreibung ohne Weiteres erkennbar und realisierbar, ohne dass der Rahmen der vorliegenden Erfindung verlassen wird.

Die vorliegende Erfindung wird anhand der folgenden Ausführungsbeispiele veranschaulicht, ohne jedoch die vorliegende Erfindung hierauf zu beschränken.

Auch wenn in den nachfolgenden Ausführungsbeispielen das erfindungsgemäße Verfahren bzw. die erfindungsgemäße Vorrichtung mit Nitrobenzol als aufzureinigendem Nitroaromaten veranschaulicht wird, ist das Verfahren bzw. die Vorrichtung nach der vorliegenden Erfindung keinesfalls darauf beschränkt, sondern ist auch auf beliebige andere Nitroaromaten, z. B. aus der Nitrierung von Toluol, Chlorbenzolen, Xylolen, Nitrobenzolen etc., und auf beliebige andere Basen als Natronlauge übertragbar.

### Ausführungsbeispiele:

### Beispiel 1: Einstufige alkalische Wäsche (Vergleichsbeispiel)

12 kg/h eines Nitrobenzols aus einer adiabatischen Nitrierung, das mit Wasser vorgewaschenen war (saure Wäsche) und das noch insgesamt 1.910 ppm an Nitrophenolen enthielt (0,8 ppm 2-Nitrophenol (2-NP), 1.346 ppm an 2,4-Dinitrophenol (2,4-DNP) und 203 ppm an 2,6-Dinitrophenol (2,6-DNP) und 360 ppm an Pikrinsäure (2,4,6-TNP)), wurde zusammen mit einer Waschlauge mit 0,8 g NaOH/l (zweifacher Überschuss, bezogen auf alle Nitrophenole) entsprechend einem Gewichtsverhältnis 1 : 1 in einen Rührkessel bei 60 °C eingespeist. Die Rührerdrehzahl wurde so eingestellt, dass im Rührkessel eine O/W-Emulsion mit dem dosierten Phasenverhältnis vorlag. Die Verweilzeit im Rührkessel betrug 6 Minuten. Nach Phasentrennung (ca. 40 Minuten) lag der pH-Wert in der Waschlauge, die 1.850 ppm an Nitrophenolen enthielt, bei ca. 11,7. Im gewaschenen Nitrobenzol wurden 60 ppm Nitrophenole gefunden. Bei Einsatz einer Waschlauge mit 4 g/l Natronlauge unter sonst gleichen Bedingungen konnte die Scheidezeit fast um den Faktor 4 auf ca. 15 Minuten verkürzt werden.

### Beispiel 2: Einstufige alkalische Wäsche (erfindungsgemäß)

12 kg/h eines Nitrobenzols aus einer adiabatischen Nitrierung, das mit Wasser vorgewaschenen war (saure Wäsche) und das noch insgesamt 1.910 ppm an Nitrophenolen enthielt (0,8 ppm 2-Nitrophenol (2-NP), 1.346 ppm an 2,4-Dinitrophenol (2,4-DNP) und 203 ppm an 2,6-Dinitrophenol (2,6-DNP) und 360 ppm an Pikrinsäure (2,4,6-TNP)), wurde mit einer Waschlauge mit 0,8 g NaOH/l (zweifacher Überschuss, bezogen auf alle Nitrophenole) im Gewichtsverhältnis 1 : 1 mittels eines Strahlmischers mit dem Waschmedium als Zentralstrahl bei 60 °C in einen Rohrreaktor eingespeist, der noch zusätzlich 5 statische Mischelemente enthielt. Die Relativgeschwindigkeit zwischen Zentralstrahl und zu waschendem Nitrobenzol betrug 8 : 1. Die Verweilzeit im Rohrreaktor betrug nicht mehr als 5 Sekunden. Der Druckabfall über die gesamte Rohrreaktorlänge betrug 1,6 bar. Nach Phasentrennung der O/W-Emulsion (ca. 40 Minuten) lag der pH-Wert in der Waschlauge, die 1.908 ppm an Nitrophenolen enthielt, bei ca. 11,6. Im gewaschenen Nitrobenzol wurden 2 ppm Nitrophenole gefunden. Bei Einsatz einer Waschlauge mit 4 g/l Natronlauge unter sonst gleichen Bedingungen konnte die Scheidezeit um den Faktor 4 auf ca. 10 Minuten verkürzt werden. Mit dem zu waschenden Nitroaromaten als Zentralstrahl im Strahlmischer wurden die gleichen Ergebnisse erzielt.

### Beispiel 3: Einstufige Neutralwäsche (erfindungsgemäß)

12 kg/h eines Nitrobenzols aus einer adiabatischen Nitrierung, das nach einer Wäsche mit Alkali (siehe z. B. Beispiel 2, alkalische Wäsche) und das noch insgesamt 2 bis 5 ppm an Nitrophenolen enthielt, wurde im Gewichtsverhältnis 1 : 1 mittels eines Strahlmischers mit Wasser als Zentralstrahl bei 60 °C in einen Rohrreaktor eingespeist, der noch zusätzlich 2 statische Mischelemente enthielt. Die Relativgeschwindigkeit zwischen Zentralstrahl und zu waschendem Nitrobenzol betrug 8 : 1. Die Verweilzeit im Rohrreaktor lag bei ca. 5 Sekunden. Der Druckabfall über die gesamte Rohrreaktorlänge betrug 0,6 bar. Nach Phasentrennung (ca. 25 Minuten) lag der pH-Wert im Waschwasser mit ca. 1,5 bis 4,5 ppm an Nitrophenolen bei ca. 9,0. Im gewaschenen Nitrobenzol wurden noch 0,5 ppm Nitrophenole gefunden. Mit dem zu waschenden Nitroaromaten als Zentralstrahl im Strahlmischer wurden die gleichen Ergebnisse erzielt.

### Beispiel 4: Einstufige alkalische Wäsche (erfindungsgemäß)

20 kg/h kg eines Nitrobenzols aus einer adiabatischen Nitrierung, das mit Wasser vorgewaschenen war (saure Wäsche) und das noch insgesamt 1.910 ppm an Nitrophenolen enthielt (0,8 ppm 2-Nitrophenol (2-NP), 1.346 ppm an 2,4-Dinitrophenol (2,4-DNP) und 203 ppm an 2,6 Dinitrophenol (2,6-DNP) und 360 ppm an Picrinsäure (2,4,6-TNP)), wurde mit 4 kg/h Waschlauge mit 4 g NaOH/l (zweifacher Überschuss, bezogen auf alle Nitrophenole) entsprechend einem Gewichtsverhältnis von Nitroaromat zu Waschlauge von 5 : 1 direkt gewaschen, wobei das Waschmedium mittels eines Strahlmischers und der zu waschende Nitroaromat bei 60 °C in einen Rohrreaktor eingespeist wurden, der noch zusätzlich 5 statische Mischelemente enthielt. Die Relativgeschwindigkeit zwischen Zentralstrahl und zu waschendem Nitrobenzol betrug 8 : 1. Die Verweilzeit im Rohrreaktor betrug nicht mehr als 5 Sekunden. Der Druckabfall über die gesamte Rohrreaktorlänge betrug 1,6 bar. Nach Phasentrennung der Emulsion vom Typ W/O (ca. 5 Minuten) lag der pH-Wert in der Waschlauge, die 9.552 ppm an Nitrophenolen enthielt, bei ca. 12,3. Im gewaschenen, noch trüben Nitrobenzol wurden ca. 8 ppm Nitrophenole gefunden.

### Beispiel 5: Einstufige Neutralwäsche (erfindungsgemäß)

20 kg/h eines Nitrobenzols aus einer adiabatischen Nitrierung, das nach einer Wäsche mit Alkali (siehe Beispiel 2, alkalische Wäsche) insgesamt noch 5 bis 8 ppm an Nitrophenolen enthielt, wurde im Gewichtsverhältnis 5 : 1 mittels eines Strahlmischers mit Wasser als Zentralstrahl bei 60 °C in einen Rohrreaktor eingespeist, der noch zusätzlich 2 statische Mischelemente enthielt. Die Relativgeschwindigkeit zwischen Zentralstrahl und zu waschendem Nitrobenzol betrug 8 : 1. Die Verweilzeit im Rohrreaktor lag bei ca. 5 Sekunden. Der Druckabfall über die gesamte Rohrreaktorlänge betrug 0,6 bar. Nach Phasentrennung (ca. 20 Minuten) lag der pH-Wert im Waschwasser mit ca. 1,5 bis 4,5 ppm an Nitrophenolen bei ca. 9,0. Im gewaschenen Nitrobenzol wurden noch 0,5 ppm Nitrophenole gefunden. Mit dem zu waschenden Nitroaromaten als Zentralstrahl im Strahlmischer wurden die gleichen Ergebnisse erzielt.

## Patentansprüche

1. Verfahren zur Entfernung von Verunreinigungen aus bei der Nitrierung von nitrierbaren aromatischen Verbindungen nach Abtrennung der Nitrierendsäure anfallenden nitrierten Rohprodukten durch Behandlung mit einem Waschmedium, **dadurch gekennzeichnet,**
**dass** (a) zunächst die nitrierten Rohprodukte mit einem Waschmedium in Kontakt gebracht werden und die nitrierten Rohprodukte und das Waschmedium ineinander verteilt werden derart, dass eine Emulsion resultiert, wobei die Herstellung der Emulsion in Schritt (a) mittels einer Dispergiereinrichtung erfolgt, und
**dass** (b) nachfolgend die resultierende Emulsion in einen Rohrreaktor einspeist wird, so dass während des Durchtritts der Emulsion durch den Rohrreaktor die in den nitrierten Rohprodukten anfänglich vorhandenen Verunreinigungen entfernt werden und/oder so dass während des Durchtritts der Emulsion durch den Rohrreaktor die in den nitrierten Rohprodukten anfänglich vorhandenen Verunreinigungen in das Waschmedium überführt und/oder hierdurch neutralisiert werden, wobei der Rohrreaktor mit Mischelementen zum Eintrag von zusätzlicher Mischenergie ausgestattet ist, wobei die Mischelemente als Bleche, als Blenden, als statische Mischer oder als Stromteiler ausgebildet sind, wobei der Druckabfall pro Mischelement 0,1 bar bis 3,0 bar beträgt.

2. Verfahren nach Anspruch 1, **dadurch gekennzeichnet,**
**dass** als Dispergiereinrichtung, insbesondere als Mischorgan, ein Rührkessel, ein Strahlmischer (Jet-Mixer) oder eine Pumpe, insbesondere eine Kreiselpumpe, eingesetzt wird.

3. Verfahren nach Anspruch 1 oder 2, **dadurch gekennzeichnet,**
**dass** als Dispergiereinrichtung, insbesondere als Mischorgan, eine Pumpe, insbesondere eine Kreiselpumpe, eingesetzt wird; oder
**dass** als Dispergiereinrichtung, insbesondere als Mischorgan, ein Strahlmischer (Jet-Mixer) eingesetzt wird, insbesondere wobei der Strahlmischer einen vorzugsweise zentralen Treibstahl und einen den Treibstrahl umgebendes Medium, insbesondere in Form eines Ringstrahls, erzeugt.

4. Verfahren nach Anspruch 2 oder 3, **dadurch gekennzeichnet,**
**dass** der Treibstrahl im Strahlmischer das Waschmedium oder aber das aufzureinigende nitrierte Rohprodukt ist und/oder dass das Verhältnis der Geschwindigkeiten zwischen dem zentralen Treibstrahl einerseits und dem den zentralen Treibstrahl umgebenden Medium, insbesondere Ringstrahl, im Strahlmischer im Bereich von 1 : 5 bis 30 : 1, bevorzugt im Bereich von 1 : 2 bis 20 : 1, besonders bevorzugt im Bereich von 1 : 1 bis 10 : 1, eingestellt wird.

5. Verfahren nach einem der Ansprüche 1 bis 4, **dadurch gekennzeichnet,**
**dass** die Dispergiereinrichtung, insbesondere das Mischorgan, dem Rohrreaktor vorgeschaltet, insbesondere unmittelbar vorgeschaltet, ist, insbesondere wobei die Dispergiereinrichtung, insbesondere das Mischorgan, in den Rohrreaktor übergeht; oder
**dass** die Dispergiereinrichtung, insbesondere das Mischorgan, in den Rohrreaktor integriert ist und/oder Bestandteil des Rohrreaktors ist.

6. Verfahren nach einem der vorangehenden Ansprüche, **dadurch gekennzeichnet,**
**dass** eine Mischenergie von 10 bis 1000 Joule/Liter, bevorzugt 10 bis 500 Joule/Liter, besonders bevorzugt 20 bis 200 Joule/Liter, eingetragen wird, insbesondere durch die Mischelemente, und/oder dass der Druckabfall pro Mischelement 0,3 bis 1,5 bar, besonders bevorzugt 0,3 bis 0,8 bar, beträgt.

7. Verfahren nach einem der vorangehenden Ansprüche, **dadurch gekennzeichnet,**
**dass** die Verweilzeit im Rohrreaktor 0,1 bis 120 Sekunden, bevorzugt 0,1 bis 60 Sekunden, besonders bevorzugt 1 bis 30 Sekunden, beträgt; und/oder
**dass** das Masseverhältnis zwischen aufzureinigenden nitrierten Rohprodukten einerseits und Waschmedium andererseits im Bereich von 200 : 1 bis 1 : 10, bevorzugt im Bereich von 100 : 1 bis 1 : 5, besonders bevorzugt im Bereich von 10 : 1 bis 1 : 2, eingestellt und/oder dass das Phasenverhältnis zwischen aufzureinigenden nitrierten Rohprodukten einerseits und Waschmedium andererseits im Bereich von 25 : 1 bis 1 : 5, insbesondere im Bereich von 10 : 1 bis 1 : 2, bevorzugt im Bereich von 5 : 1 bis 1 : 1, eingestellt wird; und/oder
**dass** das Waschmedium unter Verfahrensbedingungen, insbesondere bei Temperaturen ab 5 °C, insbesondere bei Temperaturen ab 25 °C, und Atmosphärendruck, flüssig ist und bevorzugt wässrig basiert ist, vorzugsweise Wasser ist.

8. Verfahren nach einem der vorangehenden Ansprüche, **dadurch gekennzeichnet,**
**dass** dem Waschmedium mindestens eine Base zugesetzt wird, insbesondere wobei die Base ausgewählt ist aus der Gruppe von anorganischen Hydroxiden, Carbonaten, Hydrogencarbonaten, Sulfiten, Hydrogensulfiten und Ammoniak sowie deren Mischungen oder Kombinationen, bevorzugt aus der Gruppe von Natronlauge, Kalilauge, Natriumcarbonat, Kaliumcarbonat, Natriumhydrogencarbonat, Kaliumhydrogencarbonat, Ammoniak, Ammoniumcarbonat, Natriumsulfit und Natriumhydrogensulfit sowie deren Mischungen oder Kombinationen, und/oder insbesondere wobei der Gehalt an Base im Waschmedium 0,01 bis 0,4 mol/l, bevorzugt 0,02 bis 0,2 mol/l, beträgt und/oder insbesondere wobei der Gehalt an Base im Waschmedium mindestens das Doppelte der für die Neutralisation aller als Verunreinigungen enthaltener Nitrophenole benötigten Alkalimenge beträgt.

9. Verfahren nach einem der vorangehenden Ansprüche, **dadurch gekennzeichnet,**
**dass** die aufzureinigenden nitrierten Rohprodukte unter Verfahrensbedingungen, insbesondere bei Temperaturen ab 5 °C, insbesondere bei Temperaturen ab 25 °C, und Atmosphärendruck, flüssig sind und/oder dass die aufzureinigenden nitrierten Rohprodukte aus der Nitrierung von ein- oder mehrkernigen Aromaten, insbesondere Benzol, Toluol, Xylol oder halogenierten Aromaten, insbesondere chlorierten Benzolen, stammen und/oder dass die aufzureinigenden nitrierten Rohprodukte gegebenenfalls halogenierte Mono-, Di- und Trinitroaromaten sind.

10. Verfahren nach einem der vorangehenden Ansprüche, **dadurch gekennzeichnet,**
**dass** sich Verfahrensschritt (b) eine Abtrennung der von den Verunreinigungen befreiten nitrierten Produkte vom Waschmedium, vorzugsweise in einer Phasentrenneinrichtung, insbesondere Scheideeinrichtung (Scheider), anschließt; und/oder
**dass** das aus dem Rohrreaktor austretende Gemisch aus gereinigten nitrierten Produkten und Waschmedium, insbesondere vor Abtrennung der von den Verunreinigungen befreiten nitrierten Produkte vom Waschmedium, in einen Rührkessel überführt wird; und/oder
**dass** das Waschmedium, insbesondere nach Abtrennung der von den Verunreinigungen befreiten nitrierten Produkte vom Waschmedium, rezykliert wird; und/oder
**dass** das erfindungsgemäße Verfahren zur Durchführung der sauren Wäsche und/oder der basischen Wäsche und/oder der neutralen Wäsche der nitrierten Rohprodukte eingesetzt wird.

11. Produktionsanlage zur Nitrierung nitrierbarer aromatischer Verbindungen mit nachfolgender Aufreinigung der bei der Nitrierung entstehenden nitrierten Rohprodukte,
**dadurch gekennzeichnet,**
**dass** die Produktionsanlage die folgenden Einheiten umfasst:
(a) eine Nitriereinheit zur Nitrierung aromatischer Verbindungen, insbesondere mit einem oder mehreren entsprechenden Reaktionsbehältern zur Durchführung der Nitrierreaktion(en);
(b) gegebenenfalls, in Produktionslinie stromabwärts zur Nitriereinheit angeordnet, mindestens eine Abtrenneinrichtung, insbesondere eine Scheideeinrichtung (Scheider), zur Abtrennung der Nitrierendsäure von den nitrierten Rohprodukten;
(c) in Produktionslinie stromabwärts zur Nitriereinheit und zur gegebenenfalls vorhandenen Abtrenneinrichtung angeordnet, mindestens eine Wascheinrichtung zur Durchführung einer Wäsche der nitrierten Rohprodukte, wobei die Wascheinrichtung umfasst:
- mindestens eine Dispergiereinrichtung, insbesondere mindestens ein Mischorgan, zum Inkontaktbringen und Emulgieren der aufzureinigenden nitrierten Rohprodukte einerseits und dem Waschmedium andererseits und,
- stromabwärts zur Dispergiereinrichtung angeordnet, einen Rohrreaktor zur Einspeisung der in der Dispergiereinrichtung erzeugten Emulsion von aufzureinigenden nitrierten Rohprodukten einerseits und Waschmedium andererseits, wobei der Rohrreaktor derart ausgebildet ist, dass während des Durchtritts der Emulsion durch den Rohrreaktor eine Entfernung der in den nitrierten Rohprodukten anfänglich vorhandenen Verunreinigungen ermöglicht wird und/oder dass während des Durchtritts der Emulsion durch den Rohrreaktor die in den nitrierten Rohprodukten anfänglich vorhandenen Verunreinigungen in das Waschmedium überführt und/oder hierdurch neutralisiert werden, wobei der Rohrreaktor mit Mischelementen zum Eintrag von zusätzlicher Mischenergie ausgestattet ist, wobei die Mischelemente als Bleche, als Blenden, als statische Mischer oder als Stromteiler ausgebildet sind, wobei der Druckabfall pro Mischelement 0,1 bar bis 3,0 bar beträgt;
(d) gegebenenfalls, in Produktionslinie stromabwärts zur Wascheinrichtung angeordnet, einen Rührkessel, insbesondere zur Erhöhung der Kontakt- und/oder Verweilzeit zwischen nitrierten Produkten einerseits und Waschmedium andererseits;
(e) in Produktionslinie stromabwärts zur Wascheinheit und zum gegebenenfalls vorhandenen Rührkessel angeordnet, eine Abtrenneinrichtung, insbesondere eine Scheideeinrichtung (Scheider), zur Abtrennung der von den Verunreinigungen befreiten nitrierten Produkte vom Waschmedium.

12. Produktionsanlage nach Anspruch 11, **dadurch gekennzeichnet, dass** die Dispergiereinrichtung, insbesondere das Mischorgan, ein Rührkessel, ein Strahlmischer (Jet-Mixer) oder eine Pumpe, insbesondere eine Kreiselpumpe, vorzugsweise eine Pumpe, insbesondere eine Kreiselpumpe, oder ein Strahlmischer (Jet-Mixer), besonders bevorzugt ein Strahlmischer (Jet-Mixer), ist.

13. Produktionsanlage nach Anspruch 11 oder 12, **dadurch gekennzeichnet, dass** die Dispergiereinrichtung, insbesondere das Mischorgan, dem Reaktor vorgeschaltet, insbesondere unmittelbar vorgeschaltet, ist, insbesondere wobei die Dispergiereinrichtung, insbesondere das Mischorgan, in den Rohrreaktor übergeht.

14. Produktionsanlage nach Anspruch 11 oder 12, **dadurch gekennzeichnet, dass** die Dispergiereinrichtung, insbesondere das Mischorgan, in den Rohrreaktor integriert ist und/oder Bestandteil des Rohrreaktors ist.

## Claims

1. A method for removing contaminations accumulating from nitrated raw products during the nitration of nitratable, aromatic compounds after separation of the nitrating end acid, by means of the treatment using a washing medium,
**characterized in that**
(a) initially the nitrated raw products are brought into contact with a washing medium, and the nitrated raw products and the washing medium are distributed among each other such that an emulsion results, wherein the production of the emulsion in step (a) is carried out by means of a dispersion unit, and
(b) subsequently the resulting emulsion is introduced into a tubular reactor such that during the passage of the emulsion through the tubular reactor the contaminations initially present in the nitrated raw products are removed, and/or such that during the passage of the emulsion through the tubular reactor the contaminations initially present in the nitrated raw products are transferred into the washing medium, and/or are thereby neutralized, wherein the tubular reactor is equipped with mixing members for introducing additional mixing energy, wherein the mixing members are embodied as plates, as baffles, as static mixers or as current dividers, wherein the pressure drop per mixing member is 0.1 bar to 3.0 bar.

2. The method according to claim 1, **characterized in that** an agitating vessel, a jet mixer, or a pump, in particular a rotary pump, is utilized as the dispersion unit, in particular as the mixing element.

3. The method according to claims 1 or 2, **characterized in that**
a pump, in particular a rotary pump, is utilized as the dispersion unit, in particular as the mixing element; or
a jet mixer is utilized as the dispersion unit, in particular as the mixing element, in particular wherein the jet mixer creates a preferably central propulsion jet, and a medium surrounding the propulsion jet, in particular in the form of an annular jet.

4. The method according to claims 2 or 3, **characterized in that** the propulsion jet in the rotary mixer is the washing medium, or even the nitrated raw product to be decontaminated, and/or the ratio of the speeds between the central propulsion jet on one hand, and the medium surrounding the central propulsion jet, in particular the annular jet on the other hand, in the jet mixer is adjusted to the range of 1 : 5 to 30 : 1, preferably to the range of 1 : 2 to 20 : 1, particularly preferred to the range of 1 : 1 to 10 : 1.

5. The method according to one of the claims 1 to 4, **characterized in that**
the dispersion unit, in particular the mixing element, is connected upstream of the tubular reactor, in particular connected directly upstream, in particular wherein the dispersion unit, in particular the mixing element, transitions into the tubular reactor; or
the dispersion unit, in particular the mixing element, is integrated into the tubular reactor, and/or is an integral part of the tubular reactor.

6. The method according to one of the preceding claims, **characterized in that**
a mixing energy of 10 to 1000 joules/liter, preferably 10 to 500 joules/liter, particularly preferred 20 to 200 joules/liter, are added, in particular via the mixing members, and/or the pressure drop per mixing member is 0.3 to 1.5 bar, particularly preferred 0.3 to 0.8 bar.

7. The method according to one of the preceding claims, **characterized in that**
the dwell time within the tubular reactor is 0.1 to 120 seconds, preferably 0.1 to 60 seconds, particularly preferred 1 to 30 seconds; and/or
the mass ratio between nitrated raw products to be decontaminated and the washing medium is adjusted to a range of 200 : 1 to 1 : 10, preferably to a range of 100 : 1 to 1 : 5, particularly preferred to a range of 10 : 1 to 1 : 2, and/or the phase ratio between nitrated raw products to be decontaminated and the washing medium is adjusted to a range of 25 : 1 to 1 : 5, in particular to a range of 10 : 1 to 1 : 2, preferably to a range of 5 : 1 to 1 : 1; and/or
the washing medium is liquid, and in particular aqueously based, in particular at temperatures from 5 °C, in particular at temperatures from 25 °C, and at atmospheric pressure, and is preferably water.

8. The method according to one of the preceding claims, **characterized in that** at least one base is added to the washing medium, in particular wherein the base is selected from the group of inorganic hydroxides, carbonates, hydrogen carbonates, sulfites, hydrogen sulfites, and ammonia, as well as the mixtures and combinations thereof, preferably from the group of caustic soda, caustic potash, sodium carbonate, sodium bicarbonate, potassium bicarbonate, ammoniac, ammonium carbonate, sodium sulfite, and sodium bisulfite, as well as the mixtures or combinations thereof, and/or in particular wherein the content of the base in the washing medium is 0.01 to 0.4 mol/l, preferably 0.02 to 0.2 mol/l, and/or in particular wherein the content of the base in the washing medium is at least twice the amount of alkali required for the neutralization of all nitrophenols contained as contaminations.

9. The method according to one of the previous claims, **characterized in that** the nitrated raw products to be decontaminated are liquid under process conditions, in particular at temperatures from 5 °C, in particular at temperatures from 25 °C, and atmospheric pressure, and/or that the nitrated raw products to be decontaminated originate from the nitration of mononuclear or polynuclear aromatics, in particular benzole, toluene, xylene, or halogenated aromatics, in particular chlorinated benzoles, and/or the nitrated raw products to be decontaminated are optionally halogenated mono, di, and trinitroaromatics.

10. The method according to one of the preceding claims, **characterized in that**
subsequent to process step (b) the nitrated products freed from the contaminations are separated from the washing medium, preferably in a phase separation unit, in particular a separation unit (separator), and/or
the mixture of decontaminated nitrated products and the washing medium discharged from the tubular reactor is transferred into an agitating vessel, in particular before separating the nitrated products, which have been freed from the contamination, from the washing medium; and/or
the washing medium is recycled, in particular after the separation of the nitrated products, which have been freed from the contamination, from the washing medium; and/or
the method according to the invention is utilized for carrying out an acidic wash, and/or an alkaline wash, and/or a neutral wash of the nitrated raw products.

11. A production facility for nitrating nitratable, aromatic compounds with subsequent purification of the nitrated raw products created during nitration,
**characterized in that**
the production facility comprises the following units:
(a) a nitration unit for nitrating aromatic compounds, in particular with one or more respective reaction vessels for carrying out the nitration reaction(s);
(b) optionally at least one separating unit being disposed in the production line downstream of the nitration unit, in particular a separator unit (separator) for separating the nitrating end acid from the nitrated raw products;
(c) at least one purification device being disposed in the production line downstream of the nitration unit, for carrying out the decontamination of the nitrated raw products, wherein the purification unit comprises the following:
- at least one dispersion unit, in particular at least one mixing element, for bringing into contact and emulsifying the nitrated raw products to be decontaminated and the washing medium, and
- a tubular reactor being disposed downstream of the dispersion unit for feeding the emulsion of nitrated raw products to be decontaminated, which are created in the dispersion unit on one hand, and the washing medium on the other hand, wherein the tubular reactor is embodied such that the removal of the contaminations initially present in the nitrated raw products is enabled during the passage of the emulsion through the tubular reactor, and/or that the contaminations initially present in the nitrated raw products are transferred into the washing medium during the passage of the emulsion through the tubular reactor, and/or are thereby neutralized, wherein the tubular reactor is equipped with mixing members for introducing additional mixing energy, wherein the mixing members are embodied as plates, as covers, as static mixers or as current dividers, wherein the pressure drop per mixing member is 0.1 bar to 3.0 bar;
(d) optionally, an agitating vessel disposed in the production line downstream of the washing unit, in particular in order to increase the contact and/or dwell time between nitrated products and the washing medium;
(e) a separating unit disposed in the production line downstream of the washing unit and the optionally present agitation vessel, in particular a separator unit (separator) for separating the nitrated products, which have been freed of the contaminations, from the washing medium.

12. The production facility according to claim 11, **characterized in that** the dispersion unit, in particular the mixing element, is an agitating vessel, a jet mixer, or a pump, in particular a rotary pump, preferably a pump, in particular a rotary pump, or a jet mixer, particularly preferred a jet mixer.

13. The production facility according to claims 11 or 12, **characterized in that** the dispersion unit, in particular the mixing element, is connected upstream of the reactor, in particular is connected directly upstream of the reactor, in particular wherein the dispersion unit, in particular the mixing element, transitions into the tubular reactor.

14. The production facility according to claims 11 or 12, **characterized in that** the dispersion unit, in particular the mixing element, is integrated into the tubular reactor, and/or is an integral part of the tubular reactor.

## Revendications

1. Procédé pour enlever des impuretés de produits bruts nitrés résultant de la nitration de composés aromatiques nitrables après la séparation de l'acide de nitration par traitement avec un agent de lavage,
**caractérisé en ce que**
(a) les produits bruts nitrés sont d'abord mis en contact avec un agent de lavage, et les produits bruts nitrés et l'agent de lavage sont répartis l'un dans l'autre de telle sorte qu'il en résulte une émulsion, où la réalisation de l'émulsion dans l'étape (a) s'effectue au moyen d'un dispositif de dispersion et
(b) ensuite l'émulsion résultante est introduite dans un réacteur tubulaire de telle sorte que, pendant le passage de l'émulsion à travers le réacteur tubulaire, les impuretés présentes initialement dans les produits bruts nitrés sont enlevées, et/ou de telle sorte que pendant le passage de l'émulsion à travers le réacteur tubulaire les impuretés présentes initialement dans les produits bruts nitrés sont conduites à l'agent de lavage et/ou se retrouvent de ce fait neutralisées, où le réacteur tubulaire est équipé d'éléments mélangeurs, pour l'introduction d'une énergie de mélange supplémentaire, les éléments mélangeurs étant constitués en tant que tôles, en tant qu'écrans, en tant que mélangeurs statiques ou en tant que diviseurs de flux, où la chute de pression par élément mélangeur est de 0,1 bar à 3,0 bar.

2. Procédé selon la revendication 1, **caractérisé en ce que**, en tant que dispositif de dispersion, en particulier en tant qu'organe mélangeur, il est utilisé un bac d'agitation, un mélangeur à jet (jet-mixer) ou une pompe, en particulier une pompe centrifuge.

3. Procédé selon la revendication 1 ou 2, **caractérisé en ce que**,
en tant que dispositif de dispersion, en particulier en tant qu'organe mélangeur, il est utilisé une pompe, en particulier une pompe centrifuge; ou,
en tant que dispositif de dispersion, en particulier en tant qu'organe mélangeur, il est utilisé un mélangeur à jet (jet-mixer), le mélangeur à jet produisant en particulier un jet de propulsion, de préférence central, et un agent entourant le jet de propulsion, en particulier sous la forme d'un jet annulaire.

4. Procédé selon la revendication 2 ou 3, **caractérisé en ce que**
le jet de propulsion dans le mélangeur à jet est l'agent de lavage ou bien le produit brut nitré à purifier, et/ou **en ce que** le rapport des vitesses entre le jet de propulsion central d'une part et l'agent entourant le jet de propulsion central, en particulier le jet annulaire, dans le mélangeur à jet est réglé dans la plage de 1 : 5 à 30 : 1, de préférence dans la plage de 1 : 2 à 20 : 1, de façon particulièrement préférée dans la plage 1 : 1 à 10 : 1.

5. Procédé selon une des revendications 1 à 4, **caractérisé en ce que**
le dispositif de dispersion, en particulier l'organe mélangeur, est monté en amont, en particulier directement en amont, du réacteur tubulaire, le dispositif de dispersion, en particulier l'organe mélangeur, se transformant en particulier en réacteur tubulaire; ou
**en ce que** le dispositif de dispersion, en particulier l'organe mélangeur, est intégré dans le réacteur tubulaire et/ou fait partie intégrante du réacteur tubulaire.

6. Procédé selon une des revendications précédentes, **caractérisé**
**en ce qu'**une énergie de mélange de 10 à 1 000 joules/litre, de préférence de 10 à 500 joules/litre, de façon particulièrement préférée 20 à 200 joules/litre, est introduite, en particulier par les éléments mélangeurs, et/ou **en ce que** la chute de pression par élément mélangeur est de 0,3 bar à 1,5 bar, de façon particulièrement préférée de 0,3 bar à 0,8 bar.

7. Procédé selon une des revendications précédentes, **caractérisé en ce que**
le temps de séjour dans le réacteur tubulaire est de 0,1 à 120 secondes, de préférence de 0,1 à 60 secondes, de façon particulièrement préférée de 1 à 30 secondes; et/ou
**en ce que** le rapport de masses entre des produits bruts nitrés à purifier d'une part et l'agent de lavage d'autre part est réglé dans la plage de 200 : 1 à 1 : 10, de préférence dans la plage de 100 : 1 à 1 : 5, de façon particulièrement préférée dans la plage de 10 : 1 à 1 : 2 et/ou **en ce que** le rapport de phases entre des produits bruts nitrés à purifier d'une part et l'agent de lavage d'autre part est réglé dans la plage de 25 : 1 à 1 : 5, en particulier dans la plage de 10 : 1 à 1 : 2, de préférence dans la plage de 5 : 1 à 1 : 1; et/ou
**en ce que** l'agent de lavage, dans des conditions de procédé, en particulier à des températures à partir de 5 °C, en particulier à des températures à partir de 25 °C, et à la pression atmosphérique, est liquide et est de préférence à base aqueuse, de préférence est de l'eau.

8. Procédé selon une des revendications précédentes, **caractérisé en ce qu'**au moins une base est ajoutée à l'agent de lavage, la base étant en particulier sélectionnée dans le groupe constitué d'hydroxydes inorganiques, de carbonates, d'hydrogénocarbonates, de sulfites, d'hydrogénosulfites et d'ammoniac ainsi que de leurs mélanges ou combinaisons, de préférence dans le groupe constitué de soude caustique, de potasse caustique, de carbonate de sodium, de carbonate de potassium, d'hydrogénocarbonate de sodium, d'hydrogénocarbonate de potassium, d'ammoniac, de carbonate d'ammonium, de sulfite de sodium et d'hydrogénosulfite de sodium ainsi que de leurs mélanges ou combinaisons, et/ou la teneur en base dans l'agent de lavage étant en particulier de 0,01 à 0,4 mol/l, de préférence de 0,02 à 0,2 mol/l, et/ou la teneur en base dans l'agent de lavage étant en particulier au moins le double de la quantité d'alcali nécessaire pour la neutralisation de tous les nitrophénols contenus en tant qu'impuretés.

9. Procédé selon une des revendications précédentes, **caractérisé en ce que** les produits bruts nitrés à purifier, dans des conditions de procédé, en particulier à des températures à partir de 5 °C, en particulier à des températures à partir de 25 °C, et à la pression atmosphérique, sont liquides et/ou **en ce que** les produits bruts nitrés à purifier proviennent de la nitration d'aromates à un ou plusieurs noyaux, en particulier du benzène, du toluène, du xylène, ou d'aromates halogénés, en particulier des benzènes chlorés, et/ou **en ce que** les produits bruts nitrés à purifier sont éventuellement des mono-, di- et trinitroaromates.

10. Procédé selon une des revendications précédentes, **caractérisé en ce que**
l'étape de procédé (b) est suivie d'une séparation entre les produits nitrés débarrassés des impuretés et l'agent de lavage, de préférence dans un dispositif de détachement de phases, en particulier un dispositif de séparation (séparateur); et/ou
**en ce que** le mélange sortant du réacteur tubulaire et composé de produits nitrés nettoyés et de l'agent de lavage, en particulier avant la séparation entre les produits nitrés débarrassés des impuretés et l'agent de lavage, est transféré dans un bac d'agitation; et/ou
**en ce que** l'agent de lavage, en particulier après la séparation entre les produits nitrés débarrassés des impuretés et l'agent de lavage, est recyclé; et/ou
**en ce que** le procédé selon l'invention est mis en œuvre pour la réalisation du lavage acide et/ou du lavage basique et/ou du lavage neutre des produits bruts nitrés.

11. Installation de production pour la nitration de composés aromatiques nitrables suivie d'une purification des produits bruts nitrés résultant de la nitration,
**caractérisée en ce que**
l'installation de production comprend les unités suivantes :
(a) une unité de nitration pour la nitration de composés aromatiques, en particulier avec une ou plusieurs cuves de réaction correspondantes pour la réalisation de la/des réaction(s) de nitration;
(b) éventuellement, disposé dans la ligne de production en aval de l'unité de nitration, au moins un dispositif de détachement, en particulier un dispositif de séparation (séparateur), pour la séparation entre l'acide de nitration et les produits bruts nitrés;
(c) disposé dans la ligne de production en aval de l'unité de nitration et en aval du dispositif de détachement éventuellement présent, au moins un dispositif de lavage pour la réalisation d'un lavage des produits bruts nitrés, le dispositif de lavage comprenant :
- au moins un dispositif de dispersion, en particulier au moins un organe mélangeur, pour la mise en contact et la mise en émulsion des produits bruts nitrés à purifier d'une part et de l'agent de lavage d'autre part, et
- disposé en aval du dispositif de dispersion, un réacteur tubulaire pour l'introduction de l'émulsion, produite dans le dispositif de dispersion, de produits bruts nitrés à purifier d'une part et de l'agent de lavage d'autre part, le réacteur tubulaire étant constitué de telle sorte que, pendant le passage de l'émulsion à travers le réacteur tubulaire, un enlèvement des impuretés présentes initialement dans les produits bruts nitrés est rendu possible et/ou **en ce que**, pendant le passage de l'émulsion à travers le réacteur tubulaire, les impuretés présentes initialement dans les produits bruts nitrés sont conduites à l'agent de lavage et/ou se retrouvent de ce fait neutralisées, où le réacteur tubulaire est équipé d'éléments mélangeurs, pour l'introduction d'une énergie de mélange supplémentaire, les éléments mélangeurs étant constitués en tant que tôles, en tant qu'écrans, en tant que mélangeurs statiques ou en tant que diviseurs de flux, où la chute de pression par élément mélangeur est de 0,1 bar et 3,0 bar;
(d) éventuellement disposé dans la ligne de production en aval du dispositif de lavage, un bac d'agitation, en particulier pour augmenter le temps de contact et/ou de séjour entre des produits nitrés d'une part et l'agent de lavage d'autre part;
(e) disposé dans la ligne de production en aval de l'unité de lavage et du bac d'agitation éventuellement présent, un dispositif de détachement, en particulier un dispositif de séparation (séparateur), pour la séparation entre les produits nitrés débarrassés des impuretés et l'agent de lavage.

12. Installation de production selon la revendication 11, **caractérisée en ce que** le dispositif de dispersion, en particulier l'organe mélangeur, est un bac d'agitation, un mélangeur à jet (jet-mixer) ou une pompe, en particulier une pompe centrifuge, de préférence une pompe, en particulier une pompe centrifuge, ou un mélangeur à jet (jet-mixer), de façon particulièrement préférée un mélangeur à jet (jet-mixer).

13. Installation de production selon la revendication 11 ou 12, **caractérisée en ce que** le dispositif de dispersion, en particulier l'organe mélangeur, est monté en amont, en particulier directement en amont, du réacteur, le dispositif de dispersion, en particulier l'organe mélangeur, se transformant en particulier en réacteur tubulaire.

14. Installation de production selon la revendication 11 ou 12, **caractérisée en ce que** le dispositif de dispersion, en particulier l'organe mélangeur, est intégré dans le réacteur tubulaire et/ou fait partie intégrante du réacteur tubulaire.
